(19) Europäisches Patentamt
European Patent Office
Office européen des brevets

(11) Publication number: **0 048 572**
**B1**

(12) # EUROPEAN PATENT SPECIFICATION

(45) Date of publication of patent specification: **15.07.87**

(21) Application number: **81304139.9**

(22) Date of filing: **10.09.81**

(51) Int. Cl.$^4$: **C 07 C 43/23**, C 07 C 39/17, A 61 K 31/00, C 07 C 39/42, C 07 C 39/23, C 07 C 37/00, C 07 C 49/00, C 07 C 45/00, C 07 C 47/00, C 07 C 91/23, C 07 C 131/04 // C07D209/48, C07D303/22, C07D311/20, C07D309/12

(54) **Substituted 2-hydroxyphenyl cycloalkanes and pharmaceutical compositions thereof.**

(30) Priority: **19.09.80 US 188795**

(43) Date of publication of application:
**31.03.82 Bulletin 82/13**

(45) Publication of the grant of the patent:
**15.07.87 Bulletin 87/29**

(84) Designated Contracting States:
**AT BE CH DE FR GB IT LI LU NL SE**

(56) References cited:
**FR-A-2 402 640**
**FR-M- 1 137**
**GB-A-1 464 695**
**GB-A-2 004 870**
**US-A-3 576 882**

**The file contains technical information submitted after the application was filed and *not included in this specification***

(73) Proprietor: **PFIZER INC.**
**235 East 42nd Street**
**New York, N.Y. 10017 (US)**

(72) Inventor: **Johnson, Michael Ross**
**17 Eagle Ridge Drive Gales Ferry**
**New London Connecticut (US)**
Inventor: **Melvin, Jr., Lawrence Sherman**
**7 Seabury Avenue Ledyard**
**New London Connecticut (US)**

(74) Representative: **Graham, Philip Colin Christison et al**
**Pfizer Limited Ramsgate Road**
**Sandwich, Kent CT13 9NJ (GB)**

**EP 0 048 572 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel 3-[2-hydroxy-4-(substituted)phenyl]-4-substituted)cycloalkanols, useful as analgesic agents and as anti-emetic agents; to derivatives thereof, intermediates therefor and processes for their preparation.

Despite the current availability of a number of analgesic agents, the search for new and improved agents useful for the control of broad levels of pain and accompanied by a minimum of side-effects continues. The most commonly used agent, aspirin, is of no practical value for the control of severe pain and is known to exhibit various undesirable side-effects. Other analgesic agents, such as meperidine, codeine, and morphine, possess addictive liability. The need for improved and potent analgesic agents, is, therefore, evident.

Compounds having utility as analgesics, tranquilizers, sedatives, antianxiety agents and/or as anticonvulsants, diuretics and antidiarrheal agents are described in Belgian Patents 870,404 and 870,402, and in the equivalent published British patent applications GB 2004870A and 2005257A, respectively. The former describes 3-[2-hydroxy-4-(substituted)phenyl]cycloalkanones and cycloalkanols which may have at the 4-position of the cycloalkanone or cycloalkanol residue, an alkyl, alkenyl, phenyl or phenylalkyl substituent or, at the 5-position, an alkyl substituent; while the latter discloses certain 2-(acyclic substituted) phenols; namely, 2-(hydroxyalkyl)-4-(substituted)phenols and 2-(oxoalkyl)-4-(substituted)phenols.

U.S. Patent 3,576,887, issued April 27, 1971, discloses a series of 1-(1'-hydroxy)alkyl-2-(o-hydroxyphenyl)cyclohexanes, as intermediates in the preparation of oxaphenanthrenes which exhibit central nervous system depressant properties.

U.S. Patent 3,974,157 describes 2-phenylcyclohexanones which can be substituted in the phenyl ring with up to two alkyl, hydroxy or alkoxy groups as intermediates for preparation of 1-(aminoalkyl)-2-phenylcyclohexanols useful as analgesics, local anesthetics and antiarrhythmics.

Chemical Abstracts *85*, 176952f (1976) discloses a number of 3-phenyl- and 3-phenylalkylcyclo-hexanones as intermediates for 2-aminomethyl-3-phenyl (or phenylalkyl) cyclohexanones which exhibit analgesic, sedative, antidepressant and anticonvulsant activities.

The compounds of the present invention are 3-[2-hydroxy-4-(substituted)phenyl]cycloalkanols having at the 4-position of the cycloalkanol residue a hydroxyalkyl group, which have significant advantages over analogous compounds of Belgian Patent 870404 (GB 2004870A) having an alkyl substituent at the said position.

The compounds of this invention are 3-phenyl-cycloalkanol derivatives having the formula:

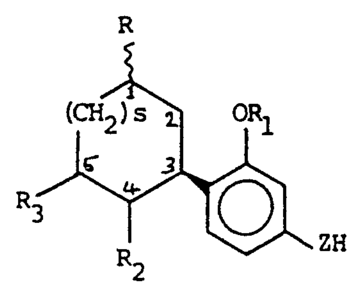

--- (I)

wherein

R is hydroxy or acetamido;
$R_1$ is hydrogen;
$R_2$ is ω-hydroxy($C_{2-4}$)alkyl or ω-methoxy($C_{2-4}$)alkyl;
$R_3$ is hydrogen or methyl;
ZH is alkyl having 7 to 11 carbon atoms; and
s is 1 or 2.

Compounds which otherwise correspond to formula I but wherein R is mandeloyloxy and/or $R_1$ is benzyl and/or $R_2$ is ω-mandeloyloxy($C_{2-4}$)alkyl are intermediates for the compounds of formula I in which R is hydroxy, $R_1$ is hydrogen or $R_2$ is ω-hydroxy($C_{2-4}$)alkyl, respectively; such intermediates are a further aspect of the invention.

Compounds of the invention contain asymmetric centres at the 1-, 3-, 4- and 5-positions in the cycloalkyl moiety and may, of course, contain additional asymmetric centres in the —ZH group attached to the phenyl ring. *Cis*-relationship between the substituent at the 1-position of the cycloalkyl moiety and the phenolic, or substituted phenolic, moiety at the 3-position is favored, and *trans*-relationship between the 3- and 4-substituents, and between the 3- and 5-substituents when $R_3$ is methyl, on the cycloalkyl moiety are favored because of the greater (quantitatively) biological activity.

For convenience, the above formulae depict the racemic compounds. However, the above formulae are considered to be generic to and embracive of the racemic modifications of the compounds of this

invention, the diastereromeric mixtures, the pure enantiomers and diastereomers thereof. The utility of the racemic mixture, the diastereomeric mixture as well as of the pure enantiomers and diastereomers is determined by the biological evaluation procedures described below.

The compounds of the invention in which R is hydroxy or mandeloyloxy are prepared by reducing a compound of the formula:

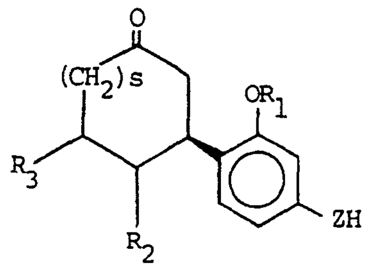

--- (II)

or a precursor thereof, to form a compound of formula I in which R is hydroxy and, if desired, acylating to form a compound in which R is mandeloyloxy and/or $R_2$ is ω-mandeloyloxy($C_{2-4}$)alkyl.

Particularly valuable compounds of the invention are those of the formulae:

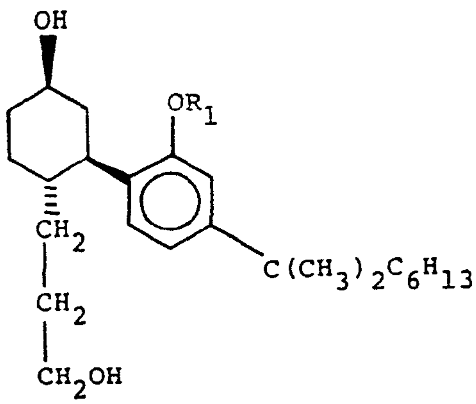

or

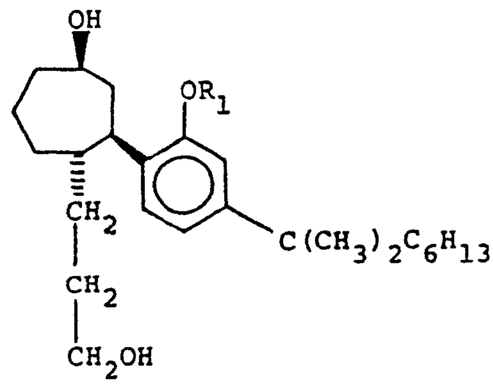

where $R_1$ is hydrogen or benzyl or a diastereomeric bis-d-mandelate thereof.

Particularly valuable intermediates for such compounds are the diastereomeric d-mandelates of compounds of the formula

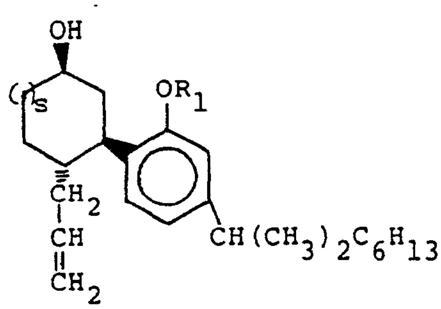

where $R_1$ is benzyl and s is 1 or 2.

Preferred compounds of the formula I are those compounds wherein:

each of $R_3$ and $R_1$ is hydrogen;

Z is $C(CH_3)_2(CH_2)_6$;

R of formula I is hydroxy (cis- and trans-forms);

$R_2$ = ω-hydroxy($C_{2-4}$)alkyl or ω-methoxy($C_{2-4}$)alkyl.

Especially preferred are compounds of formula I wherein R, $R_1$ and Z are as defined for the preferred compounds; $R_2$ is 3-hydroxypropyl or 3-methoxypropyl. The l-form of a given compound is preferred over the d-form thereof.

Particularly valuable compounds of the invention are those of the formulae:

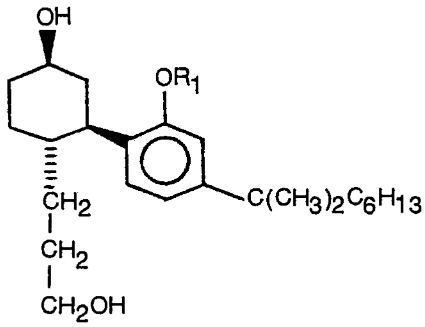

or

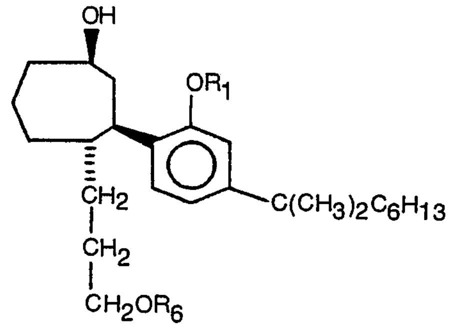

where $R_1$ is hydrogen or benzyl and $R_6$ is hydrogen or methyl, or a diastereomeric bis-d-mandelate thereof, except when $R_6$ is methyl,

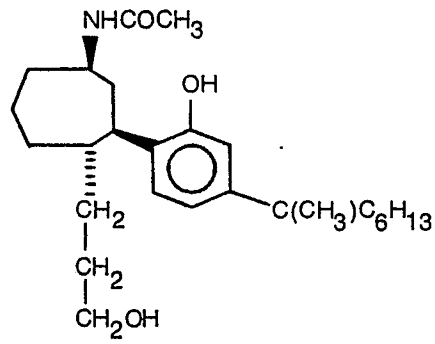

Particularly valuable intermediates for such compounds are the diastereomeric d-mandelates of compounds of the formula

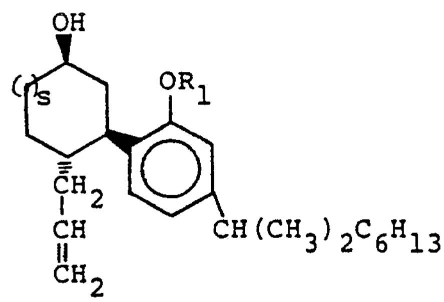

where $R_1$ is benzyl and s is 1 or 2.

The compounds of formula II are prepared from the appropriate 2-bromo-5-(Z-H substituted)phenol by a series of reactions which comprises as the first step protection of the phenolic group. Suitable protecting groups are those which do not interfere with the subsequent reactions and which can be removed under conditions which do not cause undesired reactions at other sites of said compounds or of products produced therefrom. Representative of such protective groups are those phenolic protecting groups described by Haslam in Chapter 4 of "Protective Groups in Organic Chemistry," Edited by J. F. W. McOmie, Plenum Press, London and New York (1973). Favored groups are methyl, ethyl, tetrahydropyranyl, benzyl or substituted benzyl wherein the substituent is, for example, alkyl having from one to four carbon atoms, halo (Cl, Br, F, I) and alkoxy having from one to four carbon atoms. The ether protecting, or blocking, groups can be removed through the use of hydrobromic acid in acetic acid or hydrobromic acid, 48% aqueous. The reaction is conducted at elevated temperatures and desirably at the reflux temperature. Other reagents such as hydriodic acid, pyridine hydrochloride or hydrobromide can be used to remove protecting ether groups such as methyl or ethyl groups. When the protecting groups are benzyl or substituted benzyl groups, they can be removed by catalytic hydrogenolysis. Suitable catalysts are palladium or platinum, especially when supported on carbon. Alternatively, they can be removed by solvolysis using trifluoroacetic acid. A further procedure comprises treatment with n-butyllithium in a reaction-inert solvent at room temperature.

The exact chemical structure of the protecting group is not critical to this invention since its importance resides in its ability to perform in the manner described above. The selection and identification of

appropriate protecting groups can easily and readily be made by one skilled in the art. The suitability and effectiveness of a group as a hydroxy protecting group are determined by employing such a group in the herein-illustrated reaction sequences. It should, therefore, be a group which is easily removed to regenerate the hydroxy groups. Methyl, tetrahydropyranyl and benzyl are preferred protecting groups since they are readily removed.

The protected 2-bromo-5-(Z-H-substituted)phenol is then reacted with magnesium in a reaction-inert solvent and generally in the presence of a promoter, e.g., cuprous salts such as the chloride, bromide and iodide (to promote 1,4-addition) with the appropriate 4-$R_2'$-2-cycloalken-1-one (e.g., 4-$R_2'$-2-cyclohexen-1-one) wherein $R_2'$ is alkenyl or X'-substituted alkyl wherein X' is hydroxy, oxo or a precursor therefor, e.g., -Obenzyl as precursor for —OH; —$CH(OCH_3)_2$ or —$HCOCH_2CH_2O$ as precursor for —CHO. Suitable reaction-inert solvents are cyclic and acyclic ethers such as, for example, tetrahydrofuran, dioxane and dimethyl ether of ethylene glycol (diglyme). The Grignard reagent is formed in known manner, as, for example, by refluxing a mixture of one molar proportion of the bromo reactant and two molar proportions of magnesium in a reaction-inert solvent, e.g., tetrahydrofuran. The resulting mixture is then cooled to about 0°C. to −20°C. The amount of cuprous iodide used is not critical, but can vary widely. Molar proportions ranging from about 0.2 to about 0.02 moles per mole of bromo reactant afford satisfactory yields of the cycloalkanone wherein the phenolic hydroxy group is protected (formula II, $R_1$ = a protecting group).

Alternatively, compounds of formula II are prepared by reaction of the copper derivative of an appropriate 3-(Z-H-substituted)phenol, the hydroxy group of which is suitably protected, with an appropriate 4- or 5-substituted cycloalk-2-en-1-one in a reaction-inert solvent at a low temperature, e.g., below −20°C. Suitable protecting groups are those enumerated above except, of course, benzyl or substituted benzyl which would be removed during preparation of the precursor lithio derivative of the copper 3-(Z—H)phenol. An especially useful protecting group for this alternative methodology is the tetrahydropyranyl group. Said protecting group is easily introduced into the phenol reactant, is stable to the conditions of subsequent reactions in which said protected compound serves as intermediate, and is conveniently removed by hydrolysis.

The copper derivative of the 3-(Z—H-substituted)-phenol is prepared from the lithio derivative of said phenol by treatment of said lithio derivative with 1-hexyne copper. It undergoes 1,4-addition to the conjugated C=C—C=O system of the cycloalk-2-en-1-one reactant, in contrast to the characteristic 1,2-addition of the corresponding lithio derivative.

This alternative 1,4-addition step, of course, gives rise to the same compounds as does the above described Grignard reaction. It generally affords better yields.

It will be noted that in a preparation of a compound of formula I herein wherein R is hydroxy, $R_1$ is hydrogen, $R_2$ is $(CH_2)_3OH$ and Z—H is 1,1-dimethylheptyl, the particular sequence exemplified (Examples 23—28) utilizes intermediates wherein the Z—H group is 1,1-dimethyl-2-heptenyl and which is hydrogenated in the last step of the sequence to 1,1-dimethylheptyl. Reduction of said 1,1-dimethyl-2-heptenyl group can, of course, be accomplished at any stage of the sequence if desired, i.e., it need not be delayed until the final step.

The protected cycloalkanone is then treated with an appropriate reagent to remove the protecting group, if desired. The benzyl group is conveniently removed by the method described above. If the protecting group is an alkyl group (methyl or ethyl), it is removed by the above-mentioned methods or by treatment with, for example, pyridine hydrochloride. The tetrahydropyranyl group is removed by means of an acidic reagent.

When the appropriate 4-$R_2'$-2-cycloalken-1-one is not available or is not readily obtainable by known methods, compounds of formula II wherein $R_2'$ is oxo-substituted $(C_{1-6})$alkyl, and especially those wherein $R_2'$ is formyl, serve as intermediates for all other formulae I and II compounds.

When $R_2'$ is an alkenyl group, the cycloalkanones (formula II) thus produced serve as intermediates for preparation of the corresponding cycloalkanones wherein $R_2'$ is substituted alkyl as defined above.

The cycloalkanol compounds having formula I are prepared from the protected cycloalkanones by reduction. Sodium borohydride is favored as reducing agent in this step since it not only affords satisfactory yields of the desired product, but retains the protecting group on the phenolic hydroxy group, and reacts slowly enough with hydroxylic solvents (methanol, ethanol, water) to permit their use as solvents. Temperatures of from about −40°C. to about 30°C. are generally used. Lower temperatures, even down to about −70°C., can be used to increase selectivity of the reduction. Higher temperatures cause reaction of the sodium borohydride with the hydroxylic solvent. If higher temperatures are desired, or required for a given reduction, isopropyl alcohol or the dimethyl ether of diethylene glycol are used as solvents. Sometimes favored as reducing agent is potassium tri-sec-butyl borohydride since it favors stereoselective formation of the *trans*-1,3-phenylcycloalkanol. The reduction is conducted in dry tetrahydrofuran at a temperature below about −50°C. using equimolar quantities of the ketone compound and reducing agent.

Reducing agents such as lithium borohydride, diisobutylaluminum hydride or lithium aluminum hydride which can also be used, require anhydrous conditions and non-hydroxylic solvents, such as 1,2-dimethoxyethane, tetrahydrofuran, diethyl ether, dimethyl ether of ethylene glycol.

The cycloalkanols of formula I wherein $OR_1$ is hydroxy can, of course, be obtained directly by catalytic reduction of the protected cycloalkanone over palladium-on-carbon or by catalytic reduction or chemical

reduction of the unprotected cycloalkanone (formula II, $OR_1$ = OH) using the reducing agents described above.

In actual practice it is preferred to produce the unprotected cycloalkanols of formula I ($OR_1$ = OH) via reduction of the benzyl protected cycloalkanones ($OR_1$ = benzyloxy) as described above, since it permits stereochemical control of the reduction and formation of the *cis*-hydroxy epimer as the major product and thus facilitates separation and purification of the epimeric alcohols.

Compounds wherein $R_2'$ is alkenyl are transformed to corresponding hydroxyalkyl derivatives via hydroboration-oxidation using borane in tetrahydrofuran or diethyleneglycol dimethyl ether (diglyme) at 0° to 50°C. The intermediate borane product is not isolated but is directly oxidized with alkaline hydrogen peroxide to the alcohol. The alcohols thus produced correspond to anti-Markovnikov addition of the elements of water to the double bond.

Further, hydroxyalkyl derivatives can also be produced by oxymercuration-demercuration using mercuric acetate in water followed by reduction (sodium borohydride) of the intermediate hydroxy mercurial derivative. The reaction is carried out at ambient temperature using an aqueous mercuric acetate/tetrahydrofuran medium. The intermediate is not separated, but is reduced *in situ* to the alcohol. Addition occurs opposite to that of the hydroboration-oxidation reaction; namely, Markovnikov addition, the elements of water being added to the double bond in a manner opposite to that of the hydroboration-oxidation reaction to produce secondary alcohols.

The alcohols serve as intermediates for production of corresponding aldehydes and ketones (oxoalkyl compounds) by oxidation with a suitable oxidizing agent such as pyridinium chlorochromate. The resulting oxo compounds in turn serve as intermediates for increasing the chain length of $R_2$ alkyl groups having said oxo functionality and, of course, for introducing branching into said alkyl group. The Wittig reaction, using the appropriate ylide, serves admirably for this purpose. For example, reaction of an oxoalkyl group with triphenylphosphonium methylide converts the oxo group to a methylene (=$CH_2$) group. Hydroboration-oxidation, affords a primary or secondary alcohol, itself a useful intermediate.

Formulae I and II compounds wherein $R_2'$ is oxo substituted alkyl are conveniently prepared by oxidation of corresponding compounds wherein $R_2'$ is an alkenyl group having one more carbon atom than $R_2$. Sodium metaperiodate and a catalytic amount of osmium tetroxide in aqueous tetrahydrofuran or dioxane at ambient temperature is favored as oxidizing agent since it tends to minimize oxidation beyond the aldehyde stage. However, despite its relative mildness as an oxidant compared to, for example, sodium metaperiodate/potassium permanganate, some oxidation to the corresponding acid and the corresponding hydroxy ketone does occur.

They can also be prepared by Grignard reaction of the appropriate 4-(acetal or ketal substituted alkyl)-2-cycloalken-1-one with the appropriate 2-bromo-5-(Z—H substituted)phenol according to the procedure described above. The acetal or ketal group is then transformed by treatment with acid, e.g., a mineral acid such as HCl, to the oxo group.

When transformations are to be conducted on group $R_2'$ as precursor e.g., alkenyl to hydroxyalkyl; $—CH(OCH_3)_2$ to —CHO; the desired transformations are carried out prior to removal of the protective, i.e., benzyl groups. For example, when $R_2'$ is alkenyl, said group is hydrated via the hydroboration-oxidation procedure described above, either by direct conversion from the 3-(2,4-dibenzyloxyphenyl)-4-(2-propenyl)-cycloalkanone, or by first converting the ketone to a ketal, followed by hydration of the propenyl group and then deketalization.

Formula I and II compounds wherein $R_2'$ is oxo substituted alkyl serve as intermediates for preparation of oxo substituted alkyl derivatives having increased chain length via the Wittig reaction. A representative procedure comprises, for example, reacting an appropriate 2-(1,3-dioxolan-2-yl)alkyl triphenylphosphonium bromide at about 10°C to about 80°C with sodium dimsylate in dimethyl sulfoxide to generate the corresponding ylide *in situ*. To the thus produced ylide is then added an appropriate reactant such as 3-(2-benzyloxy-4-(Z—H substituted)phenyl)-4-(2-oxoethyl)-cycloalkanone ethylene ketal in a suitable solvent such as dimethyl sulfoxide at a temperature of from about 10°C to about 80°C. The product, a 3-(2-benzyloxy-4-(Z—H substituted)phenyl)-4-(ω-oxoalk-2-enyl)cycloalkanone bis ethylene ketal, is recovered by known methods such as extraction and column chromatography on silica gel. In this Wittig reaction, the oxo group of the cycloalkanone moiety is protected by conversion to a ketal. If desired, the product is subjected to acid hydrolysis to regenerate the oxo groups. However, when the product is to be subjected to further reactions, it is advantageous to retain the bis ethylene ketal protecting groups on the product.

Reduction of the protected (ω-oxoalkenyl) group of the thus produced compounds using catalytic hydrogenation over a noble metal catalyst, e.g. Pd/C, affords the corresponding protected (ω-oxoalkyl) compound. Treatment of the reduced product with aqueous acid (HCl) as described herein provides the ω-oxoalkyl derivative of formula II wherein $R_2'$ is ω-oxoalkyl. Sodium borohydride reduction affords a formula I compound wherein $R_2$ is ω-hydroxyalkyl and R is hydroxy. The ω-hydroxyalkyl group can be converted to an ether group by the Williamson Synthesis which comprises converting the alcohol to an alkali metal alkoxide followed by alkylation of said alkoxide with an alkyl halide or alkyl sulfate.

Formation of a secondary alcohol group in $R_2'$ is readily achieved by Grignard reaction on an appropriate compound of formula II, the 1-oxo group of which is protected as an alkylene ketal, and in which $R_2'$ is oxoalkyl.

It has been found that the compound which otherwise corresponds to formula I but wherein R is OH; $R_2$ is $-(CH_2)_3OH$, —ZH is $-C(CH_3)_2(CH_2)_5CH_3$ and $R_1$ is benzyl ($C_7H_7$) can be resolved into diastereomers A and B by reaction with d-mandelic acid and p-toluenesulfonic acid in benzene with azeotropic removal of water. Hydrolysis of the bis-d-mandelate esters of diastereomers A and B (e.g., $K_2CO_3$ in methanol-water) affords enantiomers A and B.

In like manner, reaction of enantiomer B with l-mandelic acid and p-toluenesulfonic acid in benzene with azeotropic removal of water affords diastereomer A of the bis-l-mandelate derivative. Hydrolysis as described above affords enantiomer B. Removal of the protecting benzyl groups affords the corresponding enantiomeric alcohols.

Similarly, the benzyl ether of the above-mentioned compound ($R_2 = -(CH_2)_3-O-C_7H_7$) is resolved into its diastereomers and enantiomers. Other compounds of formula I herein are also resolved in like manner.

Compounds of formula I in which R is acetamido are prepared from compounds in which R is amino by reaction with acetyl chloride or acetic anhydride in the presence of a base such as pyridine.

Compounds which otherwise correspond to formula I but in which R is amino are prepared by reaction of the appropriate compound of formula I wherein R is hydroxy with equimolar quantities of phthalimide, triphenylphosphine and diethylazodicarboxylate.

If $R_2'$ is oxo-substituted alkyl and introduction of an amino group at the 1-position of the cycloalkyl moiety is desired, said oxo group must be protected as by ketal formation.

The analgesic properties of the compounds of this invention are determined by tests using nociceptive stimuli. It is noted that compounds which otherwise correspond to formula I but wherein $R_1$ is benzyl are not pharmacologically active for the purposes described herein but are useful as intermediates for said compounds wherein $R_1$ is hydrogen.

## Tests Using Thermal Nociceptive Stimuli

a) Mouse Hot Plate Analgesic Testing (=HP)

The method used is modified after Woolfe and MacDonald, *J. Pharmacol. Exp. Ther., 80,* 300—307 (1944). A controlled heat stimulus is applied to the feet of mice on a ⅛-inch thick aluminum plate. A 250 watt reflector infrared heat lamp is placed under the bottom of the aluminum plate. A thermal regulator, connected to thermistors on the plate surface, programs the heat lamp to maintain a constant temperature of 57°C. Each mouse is dropped into a glass cylinder (6½-inch diameter) resulting on the hot plate, and timing is begun when the animal's feet touch the plate. The mouse is observed at 0.5 and 2 hours after treatment with the test compound for the first "flicking" movements of one or both hind feed, or until 10 seconds elapse without such movements. Morphine has an $MPE_{50}$ = 4—5.6 mg./kg. (s.c.).

b) Mouse Tail Flick Analgesic Testing (=TF)

Tail flick testing in mice is modified after D'Amour and Smith, *J. Pharmacol. Exp. Ther., 72,* 74—79 (1941) using controlled high intensity heat applied to the tail. Each mouse is placed in a snug-fitting metal cylinder, with the tail protruding through one end. This cylinder is arranged so that the tail lies flat over a concealed heat lamp. At the onset of testing, an aluminum flag over the lamp is drawn back, allowing the light beam to pass through the slit and focus onto the end of the tail. A timer is simultaneously activated. The latency of a sudden flick of the tail is ascertained. Untreated mice usually react within 3—4 seconds after exposure to the lamp. The end point for protection is 10 seconds. Each mouse is tested at 0.5 and 2 hours after treatment with morphine and the test compound. Morphine has an $MPE_{50}$ of 3.2—5.6 mg/kg. (s.c.).

c) Tail Immersion Procedure

The method is a modification of the receptacle procedure developed by Benbasset, *et al., Arch. int. Pharmacodyn., 122,* 434 (1959). Male albino mice (19—21 g) of the Charles River CD-1 strain were weighed and marked for identification. Five animals are normally used in each drug treatment group with each animal serving as its own control. For general screening purposes, new test agents are first administered at a dose of 56 mg./kg. intraperitoneally or subcutaneously, delivered in a volume of 10 ml/kg. Preceding drug treatment and at 0.5 and 2 hours post drug, each animal is placed in the cylinder. Each cylinder is provided with holes to allow for adequate ventilation and is closed by a round nylon plug through which the animal's tail protrudes. The cylinder is held in an upright position and the tail is completely immersed in the constant temperature waterbath (56°C.). The endpoint for each trial is an energetic jerk or twitch of the tail coupled with a motor response. In some cases, the endpoint may be less vigorous post drug. To prevent undue tissue damage, the trial is terminated and the tail removed from the waterbath within 10 seconds. The response latency is recorded in seconds to the nearest 0.5 second. A vehicle control and a standard of known potency are tested concurrently with screening candidates. If the activity of a test agent has not returned to baseline values at the 2-hour testing point, response latencies are determined at 4 and 6 hours. A final measurement is made at 24 hours if activity is still observed at the end of the test day.

Test Using Chemical Nociceptive Stimuli

Suppression of Phenylbenzoquinone Irritant-Induced Writhing (=PBQ)

Groups of 5 Carworth Farms CF-1 mice are pretreated subcutaneously or orally with saline, morphine, codeine or the test compound. Twenty minutes (if treated subcutaneously) or fifty minutes (if treated orally) later, each group is treated with intraperitoneal injection of phenylbenzoquinone, an irritant known to produce abdominal contractions. The mice are observed for 5 minutes for the presence or absence of writhing starting 5 minutes after the injection of the irritant. $MPE_{50}$'s of the drug pretreatments in blocking writhing are ascertained.

Tests Using Pressure Nociceptive Stimuli

Effect on the Haffner Tail Pinch Procedure (=RTC)

A modification of the procedure of Haffner, *Experimentelle Prufung Schmerzstillender. Deuttsch Med. Wschr., 55,* 731—732 (1929) is used to ascertain the effects of the test compound on aggressive attacking responses elicited by a stimulus pinching the tail. Male albino rats (50—60 g.) of the Charles River (Sprague-Dawley) CD strain are used. Prior to drug treatment, and again at 0.5, 1, 2 and 3 hours after treatment, a Johns Hopkins 2.5-inch "bulldog" clamp is clamped onto the root of the rat's tail. The endpoint at each trial is clear attacking and biting behaviour directed toward the offending stimulus, with the latency for attack recorded in seconds. The clamp is removed in 30 seconds if attacking has not yet occurred, and the latency of response is recorded as 30 seconds. Morphine is active at 17.8 mg./kg. (i.p.).

Tests Using Electrical Nociceptive Stimuli

The "Flinch-Jump" Test (=FJ)

A modification of the flinch-jump procedure of Tenen, *Psychopharmacologia, 12,* 278—285 (1968) is used for determining pain thresholds. Male albino rats (175—200 g) of the Charles River (Sprague-Dawley) CD strain are used. Prior to receiving the drug, the feet of each rat are dipped into a 20% glycerol/saline solution. The animals are then placed in a chamber and presented with a series of 1-second shocks to the feet which are delivered in increasing intensity at 30-second intervals. These intensities are 0.26, 0.39, 0.52, 0.78, 1.05, 1.31, 1.58, 1.86, 2.13, 2.42, 2.72 and 3.04 mA. Each animal's behavior is rated for the presence of (a) flinch, (b) squeak and (c) jump or rapid forward movement at shock onset. Single upward series of shock intensities are presented to each rat just prior to, and at 0.5, 2, 4 and 24 hours subsequent to drug treatment.

Results of the above tests are recorded as percentage maximum possible effect (%MPE). The %MPE of each group is statistically compared to the %MPE of the standard and the predrug control values. The %MPE is calculated as follows:

$$\%MPE = \frac{\text{test time} - \text{control time}}{\text{cutoff time} - \text{control time}} \times 100$$

The analgesic activity of a compound is calculated as the dose (in mg/kg) which gives 50% MPE ($MPE_{50}$).

By means of the above procedures, the analgesic activity of several compounds of this invention is determined. The compounds have the formula shown below:

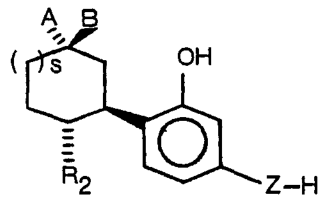

TABLE I

| | | | | | MPE$_{50}$ (mg./.kg.) | | | | |
|---|---|---|---|---|---|---|---|---|---|
| $R_2$ | A | B | s | Z—H | PBQ | RTC | HP | TF | FJ |
| $(CH_2)_2OH$ | H | OH | 1 | $C(CH_3)_2C_6H_{13}$ | 2.58 | 7.85 | | | |
| $(CH_2)_3OH$ | H | OH | 1 | $C(CH_3)_2C_6H_{13}$ | 0.29 | 0.46 | 1.11 | 0.55 | 0.63 |
| $(CH_2)_3OH^{(a)}$ | H | OH | 1 | $C(CH_3)_2C_6H_{13}$ | 0.06 | 0.13 | 0.66 | 0.41 | |
| $(CH_2)_3OH^{(b)}$ | H | OH | 1 | $C(CH_3)_2C_6H_{13}$ | 14.6 | | | | |
| $(CH_2)_4OH$ | H | OH | 1 | $C(CH_3)_2C_6H_{13}$ | 0.23 | 1.15 | | | |
| $(CH_2)_3OCH_3$ | H | OH | 1 | $C(CH_3)_2C_6H_{13}$ | 0.37 | $\sim$1.00 | | | |
| $(CH_2)_3OH$ | H | OH | 2 | $C(CH_3)_2C_6H_{13}$ | 0.1 | 0.4 | 7.8 | 1.0 | 1.0 |
| $(CH_2)_3OH$ | OH | H | 2 | $C(CH_3)_2C_6H_{13}$ | 1.11 | | $\sim$32 | 3.56 | |
| $(CH_2)_3OH^{(e)}$ | O | OH | 2 | $C(CH_3)_2C_6H_{13}$ | 0.06 | 0.1 | 0.2 | 0.2 | 0.3 |
| $(CH_2)_3OH^{(f)}$ | H | OH | 2 | $C(CH_3)_2C_6H_{13}$ | $\sim$10 | | | | |
| $(CH_2)_3OH$ | H | NHAc | 2 | $C(CH_3)_2C_6H_{13}$ | 0.2 | 1.0 | | | |

a) (−) enantiomer
b) (+) enantiomer
e) (−) enantiomer
f) (+) enantiomer

The antiemetic properties of the compounds of formula I are determined in unanesthetized unrestrained cats according to the procedure described in Proc. Soc. Exptl. Biol. and Med., *160*, 437—440 (1979).

The compounds of the present invention when used as analgesics or antiemetics vis oral and parenteral administration are conveniently administered in composition form. Such compositions include a pharmaceutical carrier selected on the basis of the chosen route of administration and standard pharmaceutical practice. For example, they may be administered in the form of tablets, pills, powders or granules containing such excipients as starch, milk sugar, certain types of clay, etc. They may be administered in capsules, in admixtures with the same or equivalent excipients. They may also be administered in the form of oral suspensions, dispersions, solutions, emulsions, syrups and elixirs which may contain flavoring and coloring agents. For oral administration of the therapeutic agents of this invention, tablets or capsules containing from about 0.01 to about 100 mg. are suitable for most applications.

The physician will determine the dosage which will be most suitable for an individual patient and it will vary with the age, weight and response of the particular patient and the route of administration. Generally, however, the initial analgesic dosage in adults may range from about 0.1 to about 750 mg per day in single or divided doses. In many instances, it is not necessary to exceed 100 mg daily. The favored oral dosage range is from about 1.0 to about 300 mg./day; the preferred dose is from about 1.0 to about 50 mg.day. The favored parenteral dose is from about 0.1 to about 100 mg/day; the preferred range from about 0.1 to about 20 mg/day.

This invention also provides pharmaceutical compositions, including unit dosage forms, valuable for the use of the herein described compounds as analgesics and other utilities disclosed herein. The dosage form can be given in single or multiple doses, as previously noted, to achieve the daily dosage effective for a particular utility.

The compounds (drugs) described herein can be formulated for administration in solid or liquid form for oral or parenteral administration. Capsules containing drugs of this invention are prepared by mixing one part by weight of drug with nine parts of excipient such as starch or milk sugar and then loading the mixture into telescoping gelatin capsules such that each capsule contains 100 parts of the mixture. Tablets containing said compounds are prepared by compounding suitable mixtures of drug and standard ingredients used in preparing tablets, such as starch, binders and lubricants, such that each tablet contains from 0.10 to 100 mg of drug per tablet.

Example 1

*Trans*-3-[2-Benzyloxy-4-(1,1-dimethylheptyl)-phenyl]-4-(2-propenyl)cyclohexanone

A solution of 73.0 g. (0.188 mole) of 1-bromo-2-benzyloxy-4-(1,1-dimethylheptyl)benzene (BrZ') in 350 ml. of tetrahydrofuran was slowly added to 9.0 g (0.375 mole) of 70—80 mesh magnesium metal. After a 5 minute initiation period the rate of addition was adjusted so as to just maintain reflux. The reaction was stirred 1.5 hours longer following completion of addition while cooling to 25°C. The reaction was cooled to −20°C. and 1.78 g (9.38 mmoles) of cuprous iodide added. The resultant mixture was stirred 5 minutes and then a solution of 25.5 g (0.188 mole) of 4-(2-propenyl)-2-cyclohexen-1-one (enone) in 30 ml of tetrahydrofuran added dropwise while the reaction temperature was maintained at about −18°C. Additional 1.78 g (9.38 mmoles) portions of cuprous iodide were added following addition of 1/3 and 2/3 of the enone reactant. The reaction was stirred 5 minutes longer at −20°C and then added to 1000 ml of ice cold saturated ammonium chloride. The quenched mixture was extracted with 1000 ml of ether and the organic extract washed twice with 500 ml of saturated ammonium chloride, once with 500 ml. of saturated sodium chloride, dried over magnesium sulfate and evaporated (aspirator) to an oil. The crude oil was purified via column chromatography on 1 kg. of silica gel eluted with 20% ether-hexane to yield 58.3 g (70%) of the title compound as an oil.

IR ($CHCl_3$) 1712, 1645, 1613 and 1575 $cm^{-1}$.

MS (m/e) 446 ($M^+$), 360, 354 and 91.

PMR $\delta^{TMS}_{CDCl_3}$ 0.82 (m, terminal methyl), 1.23 (s, gem dimethyl), 4.7—5.1 (m, vinyl H), 5.02 (s, benzylic methine), 5.3—6.1 (m, vinyl H), 6.79 (d, J=2Hz, ArH), 6.82 (dd, J=8 and 2Hz, ArH) and 7.0 (d, J=8Hz, ArH).

In like manner the following compounds were prepared from the appropriate 4-($R_2$-substituted-2-cycloalken-1-one (enone) in place of the enone used above and proportionate amounts of magnesium metal, cuprous iodide and 1-bromo-2-benzyloxy-4-(1,1-dimethylheptyl)benzene (BrR°):

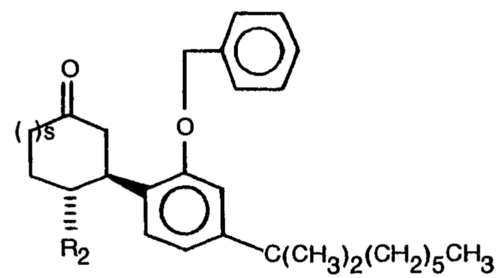

| $R_2'$ | s | Gm. Reactants Enone | Gm. Reactants BrR° | Product Yield | Product IR($CHCl_3$) $Cm^{-1}$ | Product MS (m/e) | Product PMR $\delta^{TMS}_{CDCl_3}$ |
|---|---|---|---|---|---|---|---|
| $(CH_2)_2CH=CH_2$ | 1 | 5.98 (39.9 mM) | 15.4 (39.6 mM) | 11.0 g. (60%) an oil | 1715, 1650, 1618, 1577 | 460 ($M^+$) 375, 369 | 0.81 (m, terminal methyl, 1.21 (s, gem dimethyl), 4.5—5.0 and 5.1—5.6 (m, vinyl H), 5.01 (s, benzylic methylene), 6.79 (d, J=2Hz, ArH), 6.79 (dd, J=8 and 2Hz, ArH), 7.00 (d, J=8Hz, ArH) and 7.30 (s, PhH) |
| $CH_2CH=CH_2$ | 2 | 12.7 (84.4 mM) | 50.4 (126 mM) | 25.3 g. (66%) an oil | 1702, 1650, 1618, 1580 | 454 ($M^+$) 91 | 0.80 (m, terminal methyl), 1.21 (s, gem dimethyl), 4.6—5.0 and 5.1—5.9 (m, vinyl H), 5.00 (s, benzylic methylene), 6.85 (m, ArH), 7.00 (d, J=8Hz, ArH) and 7.36 (bs, PhH) |
| $CH(OCH_3)_2$ | 1 | 10.0 (58.8 mM) | 22.9 (58.8 mM) | 14.1 g. (50%) an oil | 1721, 1626, 1584 | 480 ($M^+$) 413, 353, 329, 323, 319, 91 | 0.85 (m, terminal methyl), 1.30 (s, gem dimethyl), 3.25 and 3.26 (s, methoxys), 3.38 (m, methine), 3.96 (d, J=2Hz, ketal methine), 5.10 (s, d, J=2Hz, ketal methine), 5.10 (s, benzylic methylene), 6.9 (m, ArH), 7.10 (d, J=8Hz, ArH) and 7.42 (bs, PhH) |
| $(CH_2)_3—O—CH_2C_6H_5$ | 1 | 11.0 (45.5 mM) | 35.0 (90.0 mM) | 12.3 g. (50%) an oil | 1709, 1600, 1568 | 554.3730 ($M^+$) 463, 445, 373, 355, 337, 319, 91 (a) | 0.83 (m, terminal methyl), 1.26 (s, gem dimethyl), 1.8—2.8 and 2.8—3.5 (m), 4.40 and 5.08 (s, benzylic methylenes), 6.9 (m, ArH), 7.07 (d, J=8Hz, ArH), 7.30 and 7.38 (s, PhH) |

(a) HRMS (m/e): $M^+$ calc'd for $C_{38}H_{50}O_3$: 554.3747

Example 2

*Cis*-3-[2-Benzyloxy-4-(1,1-dimethylheptyl)phenyl]-*trans*-4-(2-propenyl)cyclohexanol and the *trans, cis* isomer

To a 0°C. solution of 14.3 g. (32.1 mmoles) of *trans*-3-[2-benzyloxy-4-(1,1-dimethylheptyl)phenyl]-4-(2-propenyl)cyclohexanone in 50 ml of methanol was added 1.22 g (32.1 mmoles) of sodium borohydride. The reaction was stirred 30 minutes at 0°C. and then added to 500 ml. saturated sodium chloride and 300 ml ether. The organic extract was washed twice with 500 ml saturated sodium chloride, dried over magnesium sulfate and evaporated (aspirator) to an oil. The crude oil was purified via column chromatography on 200 g of silica gel eluted with 2:1 pentane:ether to yield in order of elution 1.9 g (13%) of the *trans, cis*-isomer of the title compound as an oil, 2.7 g (19%) of a mixture of isomers and 7.3 g (51%) of the title compound as an oil.

*Cis-3, trans-4 isomer*:

IR ($CHCl_3$) 3571, 3401, 1639, 1610 and 1572 $cm^{-1}$.

MS (m/e) 448 ($M^+$), 406, 363 and 91.

PMR $\delta^{TMS}_{CDCl_3}$ 0.82 (m, terminal methyl), 1.22 (gem, dimethyl), 2.90 (m, benzylic methine), 3.73 (m, carbinol methine), 4.6—5.1 (m, vinyl H), 5.02 (s, benzylic methylene), 5.3—6.3 (m, vinyl H), 6.75 (d, J=2Hz, ArH), 6.75 (dd, J=8 and 2Hz, ArH), 6.99 (d, J=8Hz, ArH) and 7.25 (bs, Ph).

*Trans-3, cis-4 isomer:*

IR ($CHCl_3$) 3559, 3401, 1639, 1608 and 1567 $cm^{-1}$.

MS (m/e) 448 ($M^+$), 433, 430, 363, 406 and 91.

PMR $\delta^{TMS}_{CDCl_3}$ 0.82 (m, terminal methyl), 1.25 (s, gem dimethyl), 3.30 (m, benzylic methine), 4.12 (m, carbinol methine), 4.6—5.0 (m, vinyl H), 5.06 (s, benzylic methylene), 5.2—6.1 (m, vinyl H), 6.82 (d, J=2Hz, ArH), 6.82 (dd, J=8 and 2Hz, ArH), 7.07 (d, J=8Hz, ArH) and 7.38 (bs, Ph).

Following the above procedure, the compounds tabulated below were prepared from appropriate 3-[2-benzyloxy-4-(1,1-dimethylheptyl)phenyl]-4-(2-$R_2$-substituted)cycloalkanones and stoichiometric amounts of sodium borohydride, i.e., one gram atom/oxo group present:

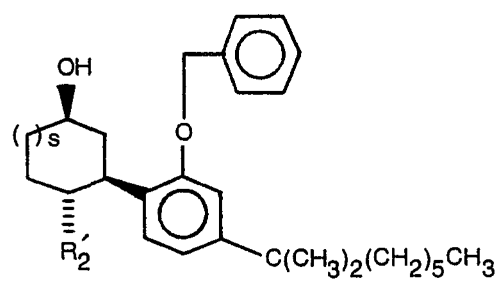

In the table, R° represents the 3-[2-benzyloxy-4-(1,1-dimethylheptyl)phenyl] moiety.

| 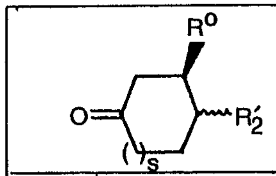 Reactant | | | | Product | | | | | |
|---|---|---|---|---|---|---|---|---|---|
| s | $R_2'$ | R° | Gms. | R° | $R_2'$ | Yield | IR ($CHCl_3$) $cm^{-1}$ | MS (m/e) | PMR $\delta_{CDCl_3}^{TMS}$ |
| 1 | $(CH_2)_2CH=CH_2$ | trans | 5.5 (12 mM) | trans | cis | 0.9 g. (16%) an oil | 3413, 1658, 1626, 1585 | 462 ($M^+$), 377, 353, 91 | 0.8 (m, terminal methyl), 1.20 (s, gem dimethyl), 3.2 (m, benzylic methine), 4.09 (m, carbinol methine), 4.6—5.0 and 5.1—6.0 (m, terminal olefin), 5.05 (s, benzylic methylene), 6.82 (d, J=2, ArH), 6.82 (dd, J=8 and 2Hz, ArH), 7.07 (d, J=8Hz, ArH) and 7.38 (m, PhH). |
| | | | | cis | trans | 2.9 g. (52%) an oil | 3367, 1656, 1623, 1582 | 462 ($M^+$), 377, 91 | 0.81 (m, terminal methyl), 1.21 (s, gem dimethyl), 2.85 (m, benzylic methine), 3.60 (m, carbinol methine), 4.5—5.0 and 5.1—6.0 (m, terminal olefin), 5.00 (s, benzylic methylene), 6.80 (m, ArH), 6.99 (d, J=8Hz, ArH) and 7.30 (bs, PhH). |

| 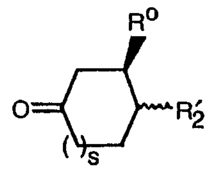 Reactant | | | | Product | | | | | |
|---|---|---|---|---|---|---|---|---|---|
| s | $R_2'$ | R° | Gms. | R° | $R_2'$ | Yield | IR ($CHCl_3$) $cm^{-1}$ | MS (m/e) | PMR $\delta_{CDCl_3}^{TMS}$ |
| 1 | $CH_2CH_2OH$ | trans | 0.900 (2.01 mM) | trans | cis | 0.130 g. (14%) an oil | 3571, 3425, 1613, 1575 | 452 ($M^+$), 432, 367, 359, 344, 343, 300, 259, 91 | |
| | | | | cis | trans | 0.770 g. (85%) an oil | 3636, 3472, 1623, 1580 | 452 ($M^+$), 432, 367, 359, 349, 343, 259, 91 | |
| 1 | $CH_2—CH(OH)—CH_3$ | trans Diastereomer A | 0.621 (1.33 mM) | trans | cis | 0.096 g. (15%) an oil | | 466 ($M^+$), 451, 448, 381, 358, 91 | |
| | | | | cis | trans | 0.425 g. (69%) an oil | 3623, 1618, 1580, | 466 ($M^+$), 451, 448, 381, 358, 274, 214, 91 | |

| 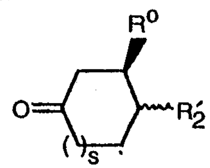 Reactant | | | | Product | | | | | |
|---|---|---|---|---|---|---|---|---|---|
| s | $R_2'$ | R° | Gms. | R° | $R_2'$ | Yield | IR ($CHCl_3$) $cm^{-1}$ | MS (m/e) | PMR $\delta_{CDCl_3}^{TMS}$ |
| | | trans | 0.895 (1.92 mM) | trans | cis | 0.079 g. (9%) an oil | | 466 ($M^+$), 451, 448, 381, 358, 273, 213 91 | |
| | | Diastereomer B | | cis | trans | 0.555 g. (62%) an oil | 3636, 3484, 1626, 1582 | 466 ($M^+$), 451, 448 381, 91 | |
| 1 | $CH(OCH_3)_2$ | trans | 35.6 (73.7 mM) | cis | trans | 14.4 g. (40%) an oil | 3521, 3389, 1600, 1562 | 482 ($M^+$), 396, 359 341, 327, 311, 91 | 0.86 (m, terminal methyl), 1.28 (s, gem dimethyl), 2.86 and 2.90 (s, methoxys), 3.75 (m, carbinol methine), 3.65 (d, J= 2Hz, ketal methine), 5.10 (s, benzylic methylene), 6.85 (m, ArH), 7.09 (d, J= 8Hz, ArH) and 7.40 (bs, PhH). |
| | | | | plus mixture of cis-trans and trans-cis isomers 6.3 g. (18%) | | | | | |

| 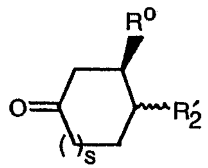 Reactant | | | | Product | | | | | |
|---|---|---|---|---|---|---|---|---|---|
| s | $R_2'$ | R° | Gms. | R° | $R_2'$ | Yield | IR ($CHCl_3$) $cm^{-1}$ | MS (m/e) | PMR $\delta^{TMS}_{CDCl_3}$ |
| 1 | $(CH_2)_3$—O—$CH_2C_6H_5$ | trans | 12.3 (50.4 mM) | trans | cis | 1.1 g. (9%) an oil | | 556.3955 ($M^+$), 448, 363 358, 273 91 (a) | 0.82 (m, terminal methyl), 1.26 (s, gem dimethyl), 3.50 (bt, J=7Hz, methylene), 4.10 (m, carbinol methine), 4.40 and 5.05 (s, benzylic methylenes), 6.84 (m, ArH), 7.05 (d, J=8Hz, ArH), 7.28 (s, PhH) and 7.38 (m, PhH). |
| | | | | cis | trans | 10.4 g. (85%) an oil | | 556.3910 ($M^+$), 505, 448, 357, 273, 91 (a) | 0.82 (m, terminal methyl), 1.25 (s, gem dimethyl), 2.8 (m, benzylic methine), 3.25 (bt, J=6Hz, methylene), 3.65 (m, carbinol methine), 4.38 and 5.07 (s, benzylic methylenes), 6.82 (d, J=2Hz, ArH), 6.82 (dd, J=8 and 2Hz, ArH), 7.02 (d, J=8Hz, ArH), 7.23 (s, PhH) and 7.35 (bs, PhH). |

(a) HRMS (m/e): $M^+$ calc'd for $C_{38}H_{52}O_3$: 556.3903

Example 3

*Trans*-3-[2-Benzyloxy-4-(1,1-dimethylheptyl)phenyl]-4-(2-hydroxyethyl)cyclohexanone ethylene ketal

Using the procedure of Example 2, 1.00 g. (2.03 mmole) of *trans*-3-[2-benzyloxy-4-(1,1-dimethylheptyl)phenyl]-4-(2-oxoethyl)cyclohexanone ethylene ketal is reduced to give a quantitative yield of the title compound as an oil.

PMR $\delta^{TMS}_{CDCl_3}$ 0.82 (m, terminal methyl), 1.11 (s, gem dimethyl), 3.50 (bt, J=6Hz, alcohol methylene), 3.91 (s, ethylene), 5.02 (s, benzylic methylene), 6.8—7.1 (m, ArH) and 7.33 (m, PhH).

Example 4

*Cis*-3-[2-Benzyloxy-4-(1,1-dimethylheptyl)phenyl]-*trans*-4-(3-hydroxypropyl)cyclohexanol

To a mechanically stirred, 0°C solution of 2.0 g (4.46 mmoles) of *cis*-3-[2-benzyloxy-4-(1,1-dimethylheptyl)phenyl-*trans*-4-(2-propenyl)cyclohexanol in 20 ml of tetrahydrofuran was added 9 ml (8.92 mmoles) of boran tetrahydrofuran complex (1M in tetrahydrofuran). The reaction was allowed to warm to 25°C and was then stirred for 30 minutes at 25°C. The reaction was cooled to 0°C and oxidized by the addition of 1 ml water, 2.66 ml (5.34 mmoles) of 2N sodium hydroxide and 0.92 ml (10.7 mmoles) of 30% hydrogen peroxide. The reaction was allowed to warm to 25°C, stirred for 35 minutes, and added to 200 ml saturated sodium chloride and 200 ml ether. The ether extract was washed twice with 100 ml of saturated sodium chloride, dried over magnesium sulfate and evaporated (aspirator) to an oil. The crude oil was purified via column chromatography on 40 g of silica gel, eluted with ether to yield 2.0 g (96%) of the title compound as an oil.

IR ($CHCl_3$) 3623, 3425, 1623 and 1580 $cm^{-1}$.

MS (m/e) 466 ($M^+$), 448, 381, 363, 358, 357 and 91.

PMR $\delta^{TMS}_{CDCl_3}$ 0.82 (m, terminal methyl), 1.24 (s, gem dimethyl), 2.90 (m, benzylic methine), 3.2—3.9 (m, carbinol methine and methylene), 5.08 (s, benzylic methylene), 6.86 (d, J=2Hz, ArH), 6.86 (dd, J=8 and 2Hz, ArH) and 7.05 (d, J=8Hz, ArH).

In like manner the following compounds are prepared from appropriate reactants.

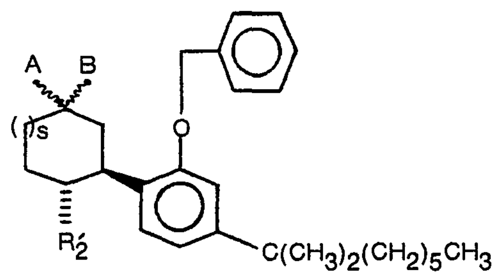

| s | Reactant | | | Product | | | | |
|---|---|---|---|---|---|---|---|---|
| s | 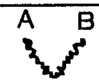 | $R_2'$ | Gms. | $R_2$ | Yield | IR ($CHCl_3$) $cm^{-1}$ | MS(m/e) | PMR $\delta_{CDCl_3}^{TMS}$ |
| 1 | 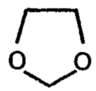 | $CH_2—CH{=}CH_2$ | 16.2 (33.1 mM) | $CH_2CH_2CH_2OH$ | Quantitative an oil | | | 0.86 (m, terminal methyl), 1.26 (s, gem dimethyl), 3.08 (m, benzylic methine), 3.47 (t, J=7Hz, alcohol methylene), 3.93 (s, ethylene), 5.10 (s, benzylic methylene), 6.90 (m, ArH), 7.08 (d, J=8Hz, ArH) and 7.42 (m, PhH). |
| 2 | 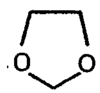 | $CH_2—CH{=}CH_2$ | 4.70 (9.32 mM) | $CH_2CH_2CH_2OH$ | Quantitative an oil | 3509, 1613, 1577, 1502, 1466 | 522 ($M^+$) | 0.83 (m, terminal methyl), 1.23 (s, gem dimethyl), 2.9, 3.7 (m, alcohol methylene and benzylic methine), 3.82 (bs, ethylene), 5.06 (benzylic methylene), 6.85 (m, ArH), 7.02. |
| | | | | | | | | (d, J=8Hz, ArH) and 7.38 (m, PhH). |
| 1 | 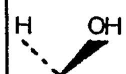 | $(CH_2)_2CH{=}CH_2$ | 3.3 (7.14 mM) | $(CH_2)_3CH_2OH$ | 2.3 g (67%) an oil | 3448, 1623, 1580 | 480 ($M^+$), 462, 395, 91 | |
| 2 | 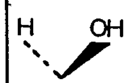 | $CH_2CH{=}CH_2$ | 1.0 (2.16 mM) | $CH_2CH_2CH_2OH$ | 1.0 g. (97%) an oil | | | 0.9 (m, terminal methyl), 1.28 (s, gem dimethyl), 3.48 (m, alcohol methylene), 3.70 (m, carbinol methylene), 5.02 (s, benzylic methylene) 6.90 (m, ArH) and 7.38 (bs, PhH). |
| 2 | 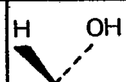 | $CH_2—CH{=}CH_2$ | 5.0 (10.8 mM) | $CH_2CH_2CH_2OH$ | 4.2 g. (82%) an oil | | | 0.80 (m, terminal methyl), 1.20 (s, gem dimethyl), 3.36 (m, alcohol methylene). |
| | | | | | | | | 4.02 (m, carbinol methine), 5.07 (s, benzylic methylene) 6.85 (m, ArH), 7.06 (d, J=8Hz, ArH) and 7.38 (s, PhH). |

| s | Reactant | | | Product | | | | |
|---|---|---|---|---|---|---|---|---|
| | 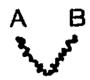 | $R_2'$ | Gms. | $R_2$ | Yield | IR ($CHCl_3$) $cm^{-1}$ | MS(m/e) | PMR $\delta_{CDCl_3}^{TMS}$ |
| 2 | 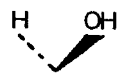 (Enantiomer A) | $CH_2CH=CH_2$ | 0.874 (1.89 mM) | $CH_2CH_2CH_2OH$ | 0.778 g. (86%) an oil | | 4.80.3561 ($M^+$), 395, 377, 372, 371, 287, 233, 91 (a) (b) | |
| 2 | 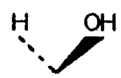 (Enantiomer B) | $CH_2CH=CH_2$ | 0.829 (1.79 mM) | $CH_2CH_2CH_2OH$ | 0.686 g. (80%) an oil | | 480.3565 ($M^+$), 395, 377, 372, 371, 287, 233, 91 (a) | |
| 1 | 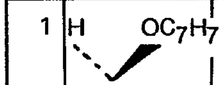 | $CH_2—CH=CH_2$ | | $CH_2CH_2CH_2OH$ | 2.09 g. (97%) an oil | 3571, 3389, 1600, 1562 | 556.3865 ($M^+$), 540, 471, 455, 358, 357, 273, 272, 257, 91 (c) | 1.22 (s, gem dimethyl), 2.78 (m, benzylic methine), 3.2—4.0 (m), 4.61 and 5.12 (s, benzylic methylene), 6.9 (m, ArH) and 7.35 (m, PhH). |

(a) High resolution mass spectral (HRMS); $M^+$ calcd. for $C_{32}H_{48}O_3$ = 480.3597
(b) $[\alpha]_D^{CH_3OH}$, 25°C = −20.57°
(c) HRMS: $M^+$ calcd. for $C_{38}H_{52}O_3$ = 556.3903.

Example 5

*Cis*-3-[4-(1,1-Dimethylheptyl)-2-hydroxyphenyl]-*trans*-4-(3-hydroxypropyl)cyclohexanol

A mixture of 2.0 g (4.29 mmole) of *cis*-3-[2-benzyloxy-4-(1,1-dimethylheptyl)phenyl]-*trans*-4-(3-hydroxypropyl)cyclohexanol and 300 mg of 5% Pd/C/50% water in 10 ml of ethanol was stirred under 1 atmosphere of hydrogen gas for 45 minutes at room temperature. The reaction was filtered through diatomaceous earth and the filtrate evaporated under reduced pressure to a solid. Recrystallization of the crude solid product from diisopropyl ether gave 1.41 g (94%) of the title compound.

m.p.: 132—3°C (diisopropyl ether).

IR (KBr) 3448, 3226, 1626 and 1592 $cm^{-1}$.

MS (m/e) 376 ($M^+$), 358, 304, 291 and 273.

PMR ($CDCl_3$, $D_2O$, $D_6$-DMSO) δ 0.83 (m, terminal methyl), 1.23 (s, gem dimethyl), 3.39 (t, J=6Hz, carbinol methylene), 3.64 (m, carbinol methine), 6.85 (m, ArH) and 6.91 (d, J=8Hz, ArH).

*Analysis:* Calc'd for $C_{24}H_{40}O_3$: C, 76.55; H, 10.71.
*Found:* C, 76.55; H, 10.44.

Similarly, the following compounds are prepared from appropriate benzyl ether reactants:

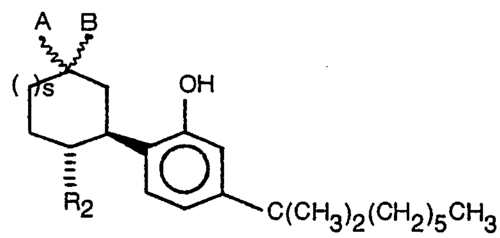

(The value of $R_2$ is the same in reactant and product).

| | Reactant | | | Product | | | |
|---|---|---|---|---|---|---|---|
| s | 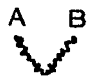 | $R_2'$ | Gms. | Yield | IR ($CHCl_3$) $cm^{-1}$ | MS (m/e) | PMR $\delta_{CDCl_3}^{TMS}$ |
| 1 | 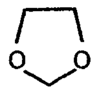 | $CH_2CH_2CH_2OH$ | 16.8 (33.1 mM) | 11.0 g. (80%) an oil | 3509, 3279, 1613, 1567 | 418 ($M^+$), 356, 333, 332, 317, 291, 271 | 0.82 (m, terminal methyl), 1.27 (s, gem dimethyl), 2.87 (m, benzylic methine), 3.50 (t, J=6Hz, alcohol methylene), 4.00 (s, ethylene), 6.8 (m, ArH) and 7.02 (d, J=8Hz, ArH). |
| 1 | 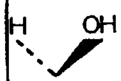 | $CH_2CH_2OH$ | 0.750 (1.66 mM) | 0.487 g. (81%) mp: 50—55°C. (semisolid from pentane) | 3636, 3356, 1631, 1587, 1577 | 362 ($M^+$), 344, 300, 290, 277, 259 | 0.85 (m, terminal methyl), 1.28 (s, gem dimethyl), 2.80 (m, benzylic methine), 3.60 (t, J=6Hz, alcohol methylene), 3.8 (m, carbinol methine), 6.80 (m, ArH) and 7.08 (d, J=8Hz, ArH). |
| 1 | 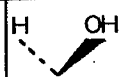 | $(CH_2)_3CH_2OH$ | 2.3 (4.79 mM) | 1.4 g (75%) mp: 125—128°C (petroleum ether) | 3390, 3247, 1634, 1597 (KBr pellet) | | 0.8 (m, terminal methyl), 1.23 (s, gem dimethyl), 2.80 (m, benzylic methine), 3.2—4.0 (m, alcohol methylene and methine), 6.70 (m, ArH) and 6.92 (d, J=8Hz, ArH). |
| 1 | 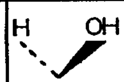 Diastereomer A | $CH_2CHCH_3$<br>\|<br>OH | 0.400 (0.858 mM) | 0.290 g (88%) an oil | 3571, 3333, 1613, 1577 | 376.2946 ($M^+$) (a) | |
| 1 | 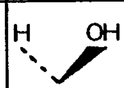 Diastereomer B | $CH_2CHCH_3$<br>\|<br>OH | 0.552 (1.12 mM) | 0.291 g. (69%) mp: 139—140°C (methylene chloride-petroleum ether) | 3534, 3268, 1613, 1563 | 376.2987 ($M^+$) (a) | |
| 1 | 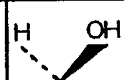 | $CH_2CHO$ | 0.432 (0.960 mM) | Quantitative an oil | 3571, 3333, 1724, 1623, 1582 | 360 ($M^+$), 342, 332, 316, 314, 298, 275, 273, 257, 247, 239, 231, 213 | 0.82 (m, terminal methyl), 1.22 (s, gem dimethyl), 2.72 (m, benzylic methine), 3.70 (m, carbinol methine), 5.60 (bs, OH), 6.65 (d, J=2Hz, ArH), 6.80 (dd, J=8 and 2Hz, ArH), 7.02 (d, J=8Hz, ArH) and 9.50 (t, J=2Hz, CHO). |

| | Reactant | | | Product | | | |
|---|---|---|---|---|---|---|---|
| s | 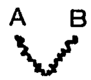 | $R_2'$ | Gms. | Yield | IR ($CHCl_3$) $cm^{-1}$ | MS (m/e) | PMR $\delta_{CDCl_3}^{TMS}$ |
| 1 | 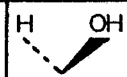 | $CH_2-C(=O)-CH_2OH$ | 0.400 (0.833 mM) | 0.313 g. (97%) a foam | 3623, 3378, 1724, 1626, 1578 | 390 ($M^+$), 372, 359, 316, 305, 298, 287, 273, 231, 213 | 0.87 (m, terminal methyl), 1.25 (s, gem dimethyl), 2.75 (m, benzylic methine), 3.70 (m, carbinol methine), 4.00 (s, hydroxy methylene), 6.80 (m, ArH) and 7.08 (d, J=8Hz, ArH). |
| 2 | =O | $CH_2CH_2CH_2OH$ | 0.960 (2.01 mM) | 0.550 g (71%) an oil | 3571, 3333, 1701, 1626 1577 | 388.3011 ($M^+$), 370, 329, 303, 285, 273, 233, 203, 161, 148, 147, 137, 137, 135 (c) | 0.82 (m, terminal methyl), (s, 1.20 (s, gem dimethyl), 3.70 (m, alcohol methylene), 6.78 (m, ArH) and 6.98 (d, J=8Hz, ArH). |
| 2 | 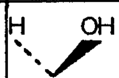 | $CH_2CH_2CH_2OH$ | 4.7 (9.79 mM) | 0.50 g (13%) mp: 114—116°C (pentane) | 3534, 3311, 1610, 1575 | 390.3136 ($M^+$), 372, 329, 305, 287, 257, 246, 233, 161, 147 (d) | 0.82 (m, terminal methyl), 2.65 (m, benzylic methine), 3.40 (m, alcohol methylene), 3.85 (m, carbinol methine), 6.15 (bs, OH), 6.75 (m, ArH) and 6.98 (d, J=8Hz, Arh). |
| 2 | 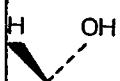 | $CH_2CH_2CH_2OH$ | 4.2 (8.75 mM) | 1.49 g (44%) mp: 57—60°C (pentane) | 3333, 1626, 1603, 1572 | 390.3132 ($M^+$), 372, 305, 287, 257, 233, 161, 147, 135 (d) | 0.80 (m, terminal methyl), 1.22 (s, gem dimethyl), 3.20 (m, benzylic methine), 3.50 (m, alcohol methylene), 4.22 (m, carbinol methine), 6.7—7.2 (m, ArH). |
| 2 | 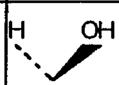 Enantiomer A | $CH_2CH_2CH_2OH$ | 0.778 (1.62 mM) | 0.466 g (74%) mp: 90—92°C (pentane) | | 390.3130 ($M^+$), 372, 305, 287, 257, 233, 161, 147 (d), (e) | |

| | Reactant | | | Product | | | |
|---|---|---|---|---|---|---|---|
| s | A B 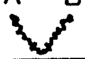 | $R_2'$ | Gms. | Yield | IR ($CHCl_3$) $cm^{-1}$ | MS (m/e) | PMR $\delta_{CDCl_3}^{TMS}$ |
| 2 | 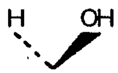 Enantiomer B | $CH_2CH_2CH_2OH$ | 0.686 (1.43 mM) | 0.447 g (80%) an oil | | 390.3095 ($M^+$), 372, 305, 287, 257, 233, 161, 147 (d), (f) | |
| 1 | 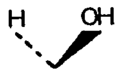 | CHO | 1.00 (2.29 mM) | 0.385 g (49%) mp: 100—102°C (ether pentane) | 3508, 3322, 1612, 1562 (g) | 346.2540 ($M^+$), 328, 261, 243 (i) | 0.82 (m, terminal methyl), 1.24 (s, gem dimethyl), 3.8 (m, carbinol methine), 5.0—5.5 (two m, hemiketal methine) and 6.8—7.2 (m, ArH). |
| 1 | 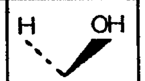 | $CH_2CH_2CH_2OH$ | 0.500 (0.847 mM) | 0.197 g (58%) mp: 72—74°C (pentane) | | 390.3100 ($M^+$), 372 286, 272, 147 (j) | 0.85 (m), 1.22 (s, gem dimethyl), 3.20 ($OCH_3$), 2.4—4.2 (several m), 5.05 (bs, OH), 6.8 (m, ArH), 7.02 (d, J=8Hz, ArH). |
| 1 | 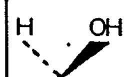 | $(CH_2)_3$—O—$CH_2C_6H_5$ (k) | 3.00 (5.40 mM) | Quantitative | 3448, 3226, 1626, 1592 | 376 ($M^+$), 358, 304, 291, 273 | |

(a) HRMS, ($M^+$) Calc'd for $C_{24}H_{40}O_3$: 376.2967
(c) HRMS, ($M^+$) Calc'd for $C_{25}H_{40}O_3$: 388.2973
(d) HRMS, ($M^+$) Calc'd for $C_{25}H_{42}O_3$: 390.3129
(e) $[\alpha]_D^{CH_3OH,}$ 25°C = −24.13°
(f) $[\alpha]_D^{CH_3OH,}$ 25°C= +23.83°
(g) Spectral data shows product exists largely in the tautomeric form but acts chemically as the product reported in the table.
(i) HRMS, ($M^+$) Calc'd for $C_{22}H_{34}O_3$: 346.2499
(j) HRMS, ($M^+$) Calc'd for $C_{25}H_{42}O_3$: 390.3123
(k) Reactant = cis-(3-[2-benzyloxy-4-[(1,1-dimethylheptyl)phenyl]-trans-4-(3-benzyloxypropyl)cyclohexanol, enantiomer A of Example 38. $R_2$ is, of course, debenzylated to $(CH_2)_3OH$.

Example 6

*Trans*-3-[2-Benzyloxy-4-(1,1-dimethylheptyl)phenyl]-4-(2-propenyl)cyclohexanone ethylene ketal

A mixture of 17.0 g (38.1 mmoles) of *trans*-3-[2-benzyloxy-4-(1,1-dimethylheptyl)phenyl]-4-(2-propenyl)cyclohexanone, 47.2 g (0.762 mole) ethylene glycol and 250 mg of *p*-toluenesulfonic acid monohydrate in 200 ml of benzene was heated at reflux for 3 hours with a Dean-Stark trap. The reaction was cooled and added to 200 ml 1*N* sodium hydroxide, 100 ml ether and 100 ml pentane. The organic extract was washed twice with 200 ml portions of water, twice with 200 ml portions of saturated sodium chloride, dried over magnesium sulfate and evaporated (aspirator) giving a quantitative yield of the title compound.

IR ($CHCl_3$) 1656, 1626 and 1587 $cm^{-1}$.

MS (m/e) 490 ($M^+$), 475, 450, 449, 448, 446, 407, 405, 399, 383 and 91.

PMR $\delta_{CDCl_3}^{TMS}$ 0.82 (m, terminal methyl), 1.22 (s, gem dimethyl), 3.1 (m, benzylic methine), 3.90 (s, ethylene ketal), 4.6—5.0 and 5.2—6.0 (m, vinyl H), 5.07 (s, benzylic methylene), 6.81 (d, J=2Hz, ArH), 6.81 (dd, J=8 and 2Hz, ArH) and 7.02 (d, J=8Hz, ArH).

In like manner, 4.5 g (9.8 mmole) of *trans*-3-[2-benzyloxy-4-(1,1-dimethylheptyl)phenyl]-4-(2-propenyl)cycloheptanone is converted in quantitative yield to *trans*-3-[2-benzyloxy-4-(1,1-dimethylheptyl)phenyl]-4-(2-propenyl)cycloheptanone ethylene ketal, an oil.

IR ($CHCl_3$) 1650, 1613, 1550, 1504 and 1460 $cm^{-1}$.

MS (m/e) 504 ($M^+$)

PMR $\delta_{CDCl_3}^{TMS}$ 0.80 (m, terminal methyl), 1.22 (s, gem dimethyl), 3.20 (m, benzylic methine), 3.80 (bs, ethylene), 4.6—6.0 (m, olefinic), 5.07 (s, benzylic methylene), 6.85 (m, ArH), 7.02 (d, J=8Hz, ArH) and 7.38 (m, PhH).

The following compounds are prepared from appropriate cycloalkanone reactants in like manner:

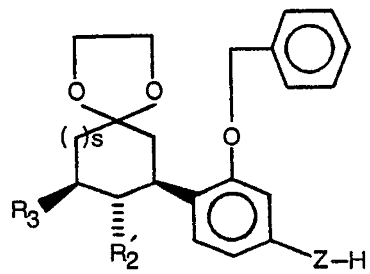

| s | $R_2'$ | $R_3$ | Z—H |
|---|---|---|---|
| 1 | $—CH_2—CH=CH_2$ | $CH_3$ | $C(CH_3)_2(CH_2)_5CH_3$ |
| 2 | $—CH_2—CH=CH_2$ | H | $C(CH_3)_2(CH_2)_5CH_3$ |

Example 7

*Trans*-3-[4-(1,1-Dimethylheptyl)-2-hydroxyphenyl]-4-(3-hydroxypropyl)cyclohexanone

A mixture of 4.0 g (9.56 mmoles) of *trans*-3-[4-(1,1-dimethylheptyl)-2-hydroxyphenyl]-4-(3-hydroxypropyl)cyclohexanone ethylene ketal, 50 ml of 2N hydrochloric acid and 100 ml of tetrahydrofuran was heated at reflux for 2 hours. The reaction was cooled and added to 500 ml saturated sodium chloride and 250 ml ether. The ether extract was separated and washed once with 500 ml saturated sodium chloride, once with 500 ml saturated sodium bicarbonate, dried over magnesium sulfate and evaporated (aspirator) to an oil. The crude oil was purified via column chromatography on 200 g of silica gel eluted with 80% ether-hexane to yield 2.93 g (82%) of the title compound as an oil.

IR ($CHCl_3$) 3521, 3333, 1709, 1616 and 1567 $cm^{-1}$.

MS (m/e) 374 ($M^+$), 356, 289, 273, 247, 203 and 161.

PMR $\delta_{CDCl_3}^{TMS}$ 0.82 (m, terminal methyl), 1.28 (s, gem dimethyl), 3.76 (m, alcohol methylene), 6.8 (m, ArH) and 6.99 (d, J=8Hz, ArH).

In like manner the following compounds are prepared from appropriate ketals:

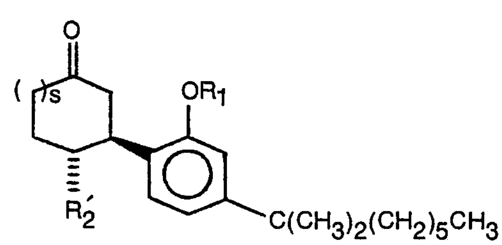

| | | | Reactant | Product | | | |
|---|---|---|---|---|---|---|---|
| s | $R_1$ | $R_2'$ | Gms. | Yield | IR ($CHCl_3$) $cm^{-1}$ | MS (m/e) | PMR $\delta_{CDCl_3}^{TMS}$ |
| 1 | $C_7H_7$ | $CH_2CH_2OH$ | 1.00<br>(2.03 mM) | Quantitative<br>an oil | 3509, 1733,<br>1626, 1587 | 450 ($M^+$),<br>365, 363,<br>359, 273,<br>91 | |
| 1 | $C_7H_7$<br>Diastereomer A | $CH_2-CH-CH_3$<br>OH | 0.680<br>(1.33 mM) | Quantitative<br>an oil | | | Rf=0.20 (0.25 mm silica gel,<br>66% ether-pentane) |
| 1 | $C_7H_7$<br>Diastereomer B | $CH_2-CH-CH_3$<br>OH | 0.980<br>(1.92 mM) | Quantitative<br>an oil | | | Rf=0.20 (0.25 mm silica gel,<br>66% ether-pentane) |
| 2 | $C_7H_7$ | $CH_2CH_2CH_2OH$ | 3.5<br>(8.1 mM) | 0.967 g<br>(31%)<br>an oil | 3571, 3425,<br>1695, 1613,<br>1577 | 478 ($M^+$) | 0.80 (m, terminal methyl), 1.27 (s, gem dimethyl), 2.7—3.6 (m, alcohol methylene and benzylic methine), 5.02 (s, benzylic methylene), 6.9 (m, ArH) and 7.4 (m, PhH). |

Example 8

*Trans*-3-[2-Benzyloxy-4-(1,1-dimethylheptyl)phenyl]-4-(2-oxoethyl)cyclohexanone ethylene ketal

A mixture of 17.0 g (34.7 mmoles) of *trans*-3-[2-benzyloxy-4-(1, 1-dimethylheptyl)phenyl]-4-(2-propenyl)cyclohexanone ethylene ketal, 44.5 g (0.208 mmole) of sodium metaperiodate and 176 mg (0.69 mmole) of osmium tetroxide in 340 ml tetrahydrofuran and 100 ml of water was stirred at room temperature for 3.5 hours. The reaction mixture was then added to 1000 ml 15% sodium sulfite-1000 ml ether. The organic phase was separated, washed twice with 500 ml of saturated sodium bicarbonate, dried over magnesium sulfate and evaporated (aspirator). The residue was purified by column chromatography on 400 g of silica gel eluted with 33—75% ether-petroleum ether to yield 10.0 g (59%) of the title compound as an oil.

IR ($CHCl_3$) 1730, 1621 and 1577 $cm^{-1}$.

MS (m/e) 492 ($M^+$), 464, 448, 407, 401, 357, 339, 332, 319, 317, 271 and 91.

PMR $\delta^{TMS}_{CDCl_3}$ 0.83 (m, terminal methyl), 1.23 (s, gem dimethyl), 3.2 (m, benzylic methine), 3.94 (s, ethylene ketal), 5.10 (s, benzylic methylene), 6.85 (dd, J—8 and 2Hz, ArH), 6.85 (d, J=2Hz, ArH), 7.07 (d, J=8Hz, ArH) and 9.57 (t, J=1.5Hz, CHO).

Further elution gave 5.53 g of a mixture of two compounds. This mixture was dissolved in 500 ml of ether and washed with four 250 ml portions of 1N sodium hydroxide. The ether phase was dried over magnesium sulfate and evaporated (aspirator) to yield 2.7 g of oil. This oil was further purified via column chromatography on 100 g of silica gel eluted with 50% ether-pentane to yield 2.16 g (12%) of *trans*-3-[2-benzyloxy-4-(1,1-dimethylheptyl)phenyl]-4-(3-hydroxy-2-oxopropyl)cyclohexanone ethylene ketal as an oil. The basic extract from above was acidified with ice cold 6N hydrochloric acid and then extracted with 500 ml of ether. The ether extract was washed twice with 200 ml water, once with 100 ml saturated sodium chloride, dried over magnesium sulfate and evaporated to give 2.4 g of oil which was purified by column chromatography on 100 g of silica gel eluted with 33% ethyl acetate-pentane to yield 1.51 g (9%) of *trans*-3-[2-benzyloxy-4-(1,1-dimethylheptyl)phenyl]-4-(2-carboxymethyl)cyclohexanone ethylene ketal.

*Trans*-3-[2-benzyloxy-4-(1,1-dimethylheptyl)phenyl]-4-(3-hydroxy-2-oxopropyl)cyclohexanone ethylene ketal:

IR ($CHCl_3$) 3484, 1724, 1613 and 1575 $cm^{-1}$.

MS (m/e) 522 ($M^+$), 491, 448, 432, 407, 358, 99, 91 and 86.

PMR $\delta^{TMS}_{CDCl_3}$ 0.82 (terminal methyl), 1.22 (s, gem dimethyl), 2.17 (m, methylene α to ketone), 2.88 (t, J=5Hz, OH), 3.82 (d, overlaps 3.90), 3.90 (s, ethylene), 5.08 (s, benzylic methylene), 6.83 (m, ArH), 7.02 (d, J=8Hz, ArH) and 7.40 (m, PhH).

*Trans*-3-[2-benzyloxy-4-(1,1-dimethylheptyl)phenyl]-4-(2-carboxymethyl)cyclohexanone ethylene ketal:

IR ($CHCl_3$) 3636—2273 (broad), 1724, 1621 and 1582 $cm^{-1}$.

MS (m/e) 508 ($M^+$), 449, 424, 418, 408, 402, 99, 91 and 86.

PMR $\delta^{TMS}_{CDCl_3}$ 0.82 (m, terminal methyl), 1.22 (s, gem dimethyl), 3.2 (m, benzylic methine), 3.93 (s, ethylene), 5.10 (s, benzylic methylene), 6.85 (m, ArH), 7.10 (d, J=8Hz, ArH) and 7.41 (m, PhH).

Similarly, oxidation of 2.60 g (5.80 mmole) of *cis*-3-[2-benzyloxy-4-(1,1-dimethylheptyl)phenyl]-*trans*-4-(2-propenyl)cyclohexanol gave 1.4 g (54%) of *cis*-3-[2-benzyloxy-4-(1,1-dimethylheptyl)phenyl]-*trans*-4-(2-oxoethyl)cyclohexanol; 409 mg (15%) of *cis*-3-[2-benzyloxy-4-(1,1-dimethylheptyl)phenyl]-*trans*-4-(3-hydroxy-2-oxopropyl)cyclohexanol and 120 mg (4.9%) of *cis*-3-[2-benzyloxy-4-(1,1-dimethylheptyl)phenyl]-*trans*-4-(carboxymethyl)cyclohexanol.

2-Oxoethyl compound:

IR ($CHCl_3$) 3610, 3425, 1623 and 1582 $cm^{-1}$.

MS (m/e) 450 ($M^+$), 422, 405, 365, 359, 342 and 91.

PMR $\delta^{TMS}_{CDCl_3}$ 0.82 (m, terminal methyl), 1.23 (s, gem dimethyl), 3.00 (m, benzylic methine), 3.70 (m, carbinol methine), 5.09 (s, benzylic methylene), 6.88 (d, J=2Hz, ArH), 6.88 (dd, J=8 and 2Hz, ArH), 7.10 (d, J=8Hz, ArH), 7.40 (s, PhH) and 9.53 (t, J=1.5Hz, CHO).

α-Hydroxy ketone:

IR ($CHCl_3$) 3448, 1721, 1618 and 1580 $cm^{-1}$.

MS (m/e) 480 ($M^+$), 466, 450, 449, 406 and 91.

PMR $\delta^{TMS}_{CDCl_3}$ 0.82 (m, terminal methyl), 1.22 (s, gem dimethyl), 3.75 (m, carbinol methine), 3.86 (bs, hydroxymethylene), 5.08 (s, benzylic methylene), 6.95 (m, ArH), 7.05 (d, J=8Hz, ArH) and 7.38 (s, PhH).

Acid:

Rf = 0.32 (0.25 mm, silica gel ether).

In like manner, 3.4-trans, 4,5-trans-3-[2-benzyloxy-4-(1,1-dimethylkeptyl)phenyl]-5-methyl-4-(2-propenyl)cyclohexanone ethylene ketal is oxidized to the corresponding 4-(2-oxoethyl)cyclohexanone derivative. Also produced are the corresponding 4-(3-hydroxy-2-oxopropyl)- and the 4-(2-carboxymethyl)cyclohexanone derivatives.

Example 9

*Trans*-3-[2-Benzyloxy-4-(1,1-dimethylheptyl)phenyl]-*cis*-4-(2-propenyl)cycloheptanol

To a refluxing solution of 3.0 g (0.0789 mole) of sodium borohydride in 50 ml of isopropanol was added a solution of 5.0 g (0.011 mole) of *trans*-3-[2-benzyloxy-4-(1,1-dimethylheptyl)phenyl]-4-(2-propenyl)cycloheptanone in 50 ml of isopropanol over a 1.5 hour period. The reaction was heated one hour longer, cooled and then added to 500 ml saturated sodium chloride. The quench was extracted twice with 800 ml saturated ether, the extracts combined, dried over magnesium sulfate and evaporated (aspirator) to an oil. The crude oil was purified via column chromatography on 500 g of silica gel eluted with 15% ether-hexane to yield in order of elution 1.78 g (36%) of the title compound, 0.55 g (11%) of mixture and 1.84 g (36%) of the cis 3-trans-4 isomer of the title compound as oils.

Cis, trans isomer:

IR ($CHCl_3$) 3333, 1626, 1600 and 1563 $cm^{-1}$.

MS (m/e) 462 ($M^+$) and 91.

PMR $\delta^{TMS}_{CDCl_3}$ 0.82 (m, terminal methyl), 1.22 (s, gem dimethyl), 2.70 (m, benzylic methine), 3.70 (m, carbinol methine), 4.5—5.0 and 5.1—6.0 (m, olefinic), 5.03 (s, benzylic methylene), 6.75 (m, ArH), 7.02 (d, J=8Hz) and 7.34 (s, PhH).

Title compound:

IR ($CHCl_3$) 3571, 1642, 1613 and 1575 $cm^{-1}$.

MS (m/e) 462 ($M^+$) and 91.

PMR $\delta^{TMS}_{CDCl_3}$ 0.80 (m, terminal methyl), 1.22 (s, gem dimethyl), 4.6—5.0 and 5.2—6.0 (m, olefinic H), 5.02 (s, benzylic methylene), 6.88 (m, ArH), 7.03 (d, J=8Hz, ArH) and 7.30 (bs, PhH).

## Example 10

Cis-3-[2-benzyloxy-4-(1,1-dimethylheptyl)phenyl]-*trans*-4-(2-propenyl)cycloheptyl *d*-mandelate Diasteromer A & B

A mixture of 2.2 g (4.76 mmole) of *cis*-3-[2-benzyloxy-4-(1,1-dimethylheptyl)phenyl-*trans*-4-(2-propenyl)cycloheptanol, 869 mg (5.72 mmole) of *d*-mandelic acid and 110 mg (0.579 mmole) of *p*-toluenesulfonic acid monohydrate in 40 ml of benzene was heated at reflux for 7 hours. Water was removed via a soxhlet extractor filled with a synthetic crystalline aluminosilicate (molecular sieve) such as those distributed by the Linde Company or the Davison Chemical Company. The reaction was stirred at 25°C for 9 hours and then added to 300 ml of saturated sodium bicarbonate and 300 ml ether. The ether phase was separated and washed once with 300 ml of saturated sodium bicarbonate, dried over magnesium sulfate and evaporated (aspirator) to an oil. The crude oil was purified via column chromatography on silica gel eluted with 15% ether-hexane to yield in order of elution 1.08 g (38%) of diastereomer A of the title compound, 0.233 g (8%) of mixture and 1.12 g of diastereomer B of the title compound as an oil.

Diastereomer A:

m.p.: 86—90°C (Methanol).

HRMS (m/e) 596.3883 ($M^+$ calc'd for $C_{40}H_{52}O_4$: 596.3852), 444, 359, 354, 313, 269 and 91.

PMR $\delta^{TMS}_{CDCl_3}$ 0.82 (m, terminal methyl), 1.22 (s, gem dimethyl), 2.90 (m, benzylic methine), 3.45 (m, ester methine), 4.5—6.0 (m, olefinic and mandelate H), 5.06 (s, benzyl ether methylene), 6.90 (m, ArH) and 7.38 (m, PhH).

$[\alpha]_D^{CH_3OH,}$ 25°C = +4.42°.

Diastereomer B:

HRMS (m/e) 596.3855 ($M^+$, calc'd for $C_{40}H_{52}O_4$: 596.3852), 354, 269, 107 and 91.

PMR $\delta^{TMS}_{CDCl_3}$ 0.82 (m, terminal methyl), 1.21 (s, gem dimethyl), 2.80 (m, benzylic methine), 3.40 (m, ester methine), 4.5—6.0 (m, olefinic and mandelate H), 6.80 (m, ArH) and 7.25 (m, PhH).

$[\alpha]_D^{CH_3OH,}$ 25°C = +53.34°.

## Example 11

*Cis*-3-[2-Benzyloxy-4-(1,1-dimethylheptyl)phenyl]-*trans*-4-(2-propenyl)cycloheptanol Enantiomer A

A mixture of 1.25 g (2.09 mmole) of *cis*-3-[2-benzyloxy-4-(1,1-dimethylheptyl)phenyl]-*trans*-4-(2-propenyl)cycloheptyl *d*-mandelate, diastereomer A, and 577 mg (4.18 mmole) of potassium carbonate in 20 ml methanol, 5 ml tetrahydrofuran and 2 ml of water was stirred at 25°C for 20 hours. The reaction was added to 300 ml water-250 ml ether. The ether extract was washed once with 300 ml saturated sodium chloride, dried over magnesium sulfate and evaporated. The crude product was purified via column chromatography on 75 g of silica gel eluted with 33% ether-hexane to yield 650 mg (67%) of the title compound as an oil.

HRMS (m/e) 462.3482 ($M^+$, calc'd for $C_{32}H_{46}O_2$: 462.3490), 377, 313, 269, 233, 227 and 91.

$[\alpha]_D^{CH_3OH,}$ 25°C = −20.18°.

In like manner 1.25 g (2.09 mmole) of *cis*-3-[2-benzyloxy-4-(1,1-dimethylheptyl)phenyl]-*trans*-4-(2-propenyl)cycloheptyl *d*-mandelate, diastereomer B, was converted to 383 mg (40%) of *cis*-3-[2-benzyloxy-4-(1,1-dimethylheptyl)phenyl]-*trans*-4-(2-propenyl)cycloheptanol enantiomer B as an oil.

HRMS (m/e) 462.3543 ($M^+$, calc'd for $C_{32}H_{46}O_2$: 462.3490), 377, 313, 269, 233, 227 and 91.

$[\alpha]_D^{CH_3OH}$, 25°C = +16.06°.

Example 12

*Cis*-3-[4-(1,1-Dimethylheptyl)-2-hydroxyphenyl]-*trans*-4-(3-methoxypropyl)cyclohexanol

To a slurry of 172 mg (7 mmole) of sodium hydride in 15 ml dimethylformamide was added 2.0 g (3.60 mmole) of *cis*-3-[2-benzyloxy-4-(1,1-dimethylheptyl)phenyl]-*trans*-4-(3-hydroxypropyl)-1-benzyloxycyclohexane in 15 ml dimethylformamide. The reaction mixture was then heated at 40—50°C for 3 hours, then cooled to 25°C and 0.663 ml (7.0 mmole) of dimethylsulfate was added. The resultant mixture was stirred 18 hours at 0°C and then added to 250 ml saturated sodium chloride and 250 ml ether. The organic extract was dried over magnesium sulfate and evaporated to an oil. The crude oil was purified via column chromatography on 75 g of silica gel eluted with 20% ether-hexane to yield 500 mg (24%) of *cis*-3-[4-(1,1-dimethylheptyl)-2-hydroxyphenyl)-*trans*-4-(3-methoxypropyl)cyclohexanol as an oil.

PMR $\delta^{TMS}_{CDCl_3}$ 0.83 (m, terminal methyl), 1.23 (s, gem dimethyl), 3.20 (s, $OCH_3$), 2.6—4.0 (m), 4.54 and 5.06 (s, benzylic methylenes), 6.85 (m, ArH) and 7.28 (m, PhH).

Debenzylation of 500 mg (0.874 mmole) of the product according to the procedure of Example 5 gives the title compound.

m.p.: 72°—74°C (pentane).

MS (m/e) 390.3100 ($M^+$, calc'd for $C_{25}H_{42}O_3$: 390.3123), 372, 286, 272 and 147.

PMR ($CDCl_3$) δ 0.85 (m), 1.22 (s, gem dimethyl), 3.20 ($OCH_3$), 2.4—4.2 (several m), 5.05 (bs, OH), 6.8 (m, ArH) and 7.02 (d, J=8Hz, ArH).

Example 13

*Cis*-3-[2-Benzyloxy-4-(1,1-dimethylheptyl)phenyl]-*trans*-4-(2-propenyl)-1-phthalimidocycloheptane

To a 25°C mixture of 1.00 g (2.16 mmole of *trans*-4-[2-benzyloxy-4-(1,1-dimethylheptyl)phenyl]-*cis*-4-(2-propenyl)cycloheptanol, 476 mg (3.24 mmole) of phthalimide and 846 mg (3.24 mmole) of triphenylphosphine in 1.0 ml acetonitrile was slowly added 0.51 ml (3.24 mmole) of diethyl azodicarboxylate. The reaction mixture was stirred 5 hours and then dissolved in 250 ml ether. The ether phase was washed once with 250 ml water, once with 250 ml saturated sodium chloride, dried over magnesium sulfate and evaporated to an oil. The crude oil was purified via column chromatography on 200 g of silica gel eluted with 10% ether-hexane to yield 820 mg (64%) of the title compound as an oil.

IR ($CHCl_3$) 1761, 1695, 1625, 1603 and 1563.

HRMS (m/e) 591.3799 ($M^+$, calc'd for $C_{40}H_{49}NO_3$: 591.3700), 506, 353 and 91.

PMR $\delta^{TMS}_{CDCl_3}$ 0.8 (m, terminal methyl), 1.20 (s, gem dimethyl), 2.98 (m, benzylic methine), 4.4 (bm, methine), 4.5—5.0 (m, vinyl H), 5.03 (s, benzylic methylene), 5.2—6.0 (m, vinyl H), 6.78 (m, ArH), 7.02 (d, J=8Hz, ArH), 7.33 and 7.65 (m, ArH and PhH).

Example 14

*Cis*-3-[2-Benzyloxy-4-(1,1-dimethylheptyl)phenyl]-*trans*-4-(2-propenyl)-1-aminocycloheptane

A solution of 1.0 g (1.68 mmole) of *cis*-3-[2-benzyloxy-4-(1,1-dimethylheptyl)phenyl]-*trans*-4-(2-propenyl)-1-phthalimidocycloheptane and 0.834 ml (1.68 mmole); of hydrazine hydrate in 2 ml ethanol was heated at reflux for 20 minutes forming a precipitate. The reaction was cooled, filtered and the solid washed witgh 200 ml ether. The filtrate was washed once with 100 ml saturated sodium chloride, once with 100 ml 50% 2N sodium hydroxide, dried over magnesium sulfate and evaporated to yield 770 mg (99%) of the title compound as an oil.

IR ($CHCl_3$) 3125, 1695, 1632, 1600 and 1562 $cm^{-1}$.

HRMS (m/e) 461.3607 ($M^+$, calc'd for $C_{32}H_{47}NO$: 461.3646), 420, 370, 354 and 91.

PMR $\delta^{TMS}_{CDCl_3}$ 0.8 (m, terminal methyl), 1.24 (s, gem dimethyl), 2.9 (m, 2H), 4.5—5.0 (m, vinyl H), 5.04 (s, benzylic methylene), 5.2—6.1 (m, vinyl H), 6.85 (m, ArH), 7.07 (d, J=8Hz, ArH) and 7.4 (m, PhH).

Following the procedures of Example 13 and of this example, cis-3-[2-benzyloxy-4-(1,1-dimethylheptyl)phenyl]-trans-4-(2-propenyl)-1-aminocyclohexane is prepared from trans-3-[2-benzyloxy-4-(1,1-dimethylheptyl)phenyl]-cis-4-(2-propenyl)cyclohexanol.

Example 15

*Cis*-3-[2-Benzyloxy-4-(1,1-dimethylheptyl)phenyl-*trans*-4-(2-propenyl)-1-acetamidocycloheptane

A mixture of 500 mg (1.08 mmole) of *cis*-3-[2-benzyloxy-4-(1,1-dimethylheptyl)phenyl]-*trans*-4-(2-propenyl)-1-aminocycloheptane, 131 mg (1.08 mmole) 4-N,N-dimethylaminopyridine and 0.101 ml (1.08 mmole) of acetic anhydride in 5 ml dichloromethane was stirred for one hour. The reaction was added to 100 ml ether and washed twice with 100 ml 1N hydrochloric acid and twice with 100 ml saturated sodium bicarbonate. The organic phase was dried over magnesium sulfate and evaporated to give 485 mg (89%) of the title compound as an oil.

IR ($CHCl_3$) 3389, 1652, 1602 and 1562 $cm^{-1}$.

HRMS (m/e) 503.3793 ($M^+$, calc'd for $C_{34}H_{49}NO_2$: 503.3751), 418, 412, 353 and 91.

PMR $\delta^{TMS}_{CDCl_3}$ 0.82 (m, terminal methyl), 1.22 (s, gem dimethyl), 1.86 (s, $COCH_3$), 2.95 (m, benzylic methine), 4.02 (bm, CHN), 4.6—5.0 (m, vinyl H), 5.08 (s, benzylic methylene), 5.3—6.0 (m, vinyl H), 6.85 (m, ArH), 7.03 (d, J=8Hz, ArH) and 7.38 (m, ArH).

Example 16

*Cis*-3-[2-Benzyloxy-4-(1,1-dimethylheptyl)phenyl]-*trans*-4-(3-hydroxypropyl)-1-acetamidocycloheptane

Using the procedure of Example 4, 1.7 g (3.37 mmole) of *cis*-3-[2-benzyloxy-4-(1,1-dimethylheptyl)phenyl]-*trans*-4-(2-propenyl)-1-acetamidocycloheptane gave 962 mg (55%) of the title compound as an oil.

IR ($CHCl_3$) 3448, 1652, 1600 and 1562 $cm^{-1}$.

HRMS (m/e) 521.3813 ($M^+$, calc'd for $C_{34}H_{51}NO_3$: 521.3856), 430, 371 and 91.

PMR $\delta^{TMS}_{CDCl_3}$ 0.8 (m, terminal methyl), 1.20 (s, gem dimethyl), 2.85 (m, benzylic methine), 3.35 (m, $CH_2OH$), 4.0 (m, CHN), 5.05 (s, benzylic methylene), 5.38 (bd, NH), 6.8 (m, ArH), 7.00 (d, J=8Hz, ArH) and 7.39 (bs, PhH).

Example 17

*Cis*-3-[4-(1,1-Dimethylheptyl)-2-hydroxyphenyl]-*trans*-4-(3-hydroxypropyl)-1-acetamidocycloheptane

Using the procedure of Example 5, 960 mg (1.84 mmole) of *cis*-3-[2-benzyloxy-4-(1,1-dimethylheptyl)phenyl]-*trans*-4-(3-hydroxypropyl)-1-acetamidocycloheptane gave 630 mg (79%) of the title compound as an oil.

IR ($CHCl_3$) 3649, 3389, 3279, 1652, 1600 and 1562 $cm^{-1}$.

HRMS (m/e) 431.3420 ($M^+$, calc'd for $C_{27}H_{45}NO_3$: 431.3388), 346, 287, 257, 161, 147 and 133.

PMR $\delta^{TMS}_{CDCl_3}$ 0.8 (m, terminal methyl), 1.20 (s, gem dimethyl), 1.85 (s, $COCH_3$), 2.82 (m, benzylic methine), 3.42 (m, $CH_2OH$), 4.0 (m, CHN), 5.6 (bd, NH), 6.75 (m, ArH) and 6.98 (d, J=8Hz, ArH).

By means of the procedures of Examples 15, 16 and this example, cis-3-[4-(1,1-dimethylheptyl)-2-hydroxyphenyl]-trans-(3-hydroxypropyl)-1-acetamidocyclohexane is prepared from cis-3-[2-benzyloxy-4-(1,1-dimethylheptyl)phenyl]-trans-(2-propenyl)-1-aminocyclohexane.

Example 18

*Cis*-3-[2-Benzyloxy-4-(1,1-dimethylheptyl)phenyl]-*trans*-4-(3-hydroxypropyl)cyclohexanol bis-*d*-mandelate

A mixture of 31.3 g (67.2 mmole) of *cis*-3-[2-benzyloxy-4-(1,1-dimethylheptyl)phenyl]-*trans*-4-(3-hydroxypropyl)cyclohexanol, 25.5 g (168 mmole) of *d*-mandelic acid and 1.0 g (5.26 mmole) of *p*-toluenesulfonic acid monohydrate in 500 ml of benzene was heated at reflux for 11 hours. Water was removed by use of a soxhlet extractor filled with 200 g of 3A molecular sieves. The reaction is cooled, added to one liter saturated sodium bicarbonate and extracted with four 500 ml portions of ether. The combined organic extract was dried over magnesium sulfate and evaporated to an oil. The crude oil was purified via column chromatography on 2 kg of silica gel eluted with 40—50% ether-hexane to yield in order of elution 18 g (37%) of diastereomer A and 21 g (43%) of diastereomer B of the title compound.

Diastereomer A

m.p.: 111—112.5°C (methanol).

$[\alpha]_D^{25°}$ = +20.19 (C=1.107, 5% chloroform-methanol).

PMR ($CDCl_3$) δ 0.84 (m, terminal methyl), 1.25 (s, gem dimethyl), 2.8 (m, benzylic methine), 3.45 (m), 4.0 (m), 4.6—5.2 (m), 5.05 (s, benzylic methylene), 6.88 (m, ArH), 7.27 and 7.34 (s, PhH).

| | | |
|---|---|---|
| *Analysis:* Calc'd for $C_{47}H_{58}O_7$: | C, 76.81; | H, 7.95. |
| *Found:* | C, 76.49; | H, 7.76. |

In like manner, but using the same relative stoichiometric proportion of d-mandelic acid and p-toluenesulfonic acid monohydrate, 9.0 g (16 mmole) of *cis*-3-[2-Benzyloxy-4-(1,1-dimethylheptyl)phenyl]-*trans*-4-(3-benzyloxypropyl)cyclohexanol gave, in order of elution, 4.1 g (37%) of diastereomer A and 4.2 g (38%) of diastereomer B of *cis*-3-[2-benzyloxy-4-(1,1-dimethylheptyl)phenyl]-*trans*-4-(3-benzyloxypropyl)-1-*d*-mandeloyloxycyclohexane as solids.

Diastereomer A:

m.p.: 56—58°C.

$[\alpha]_D^{25°}$ = 0.292° (C = 1.25, $CH_3OH$).

PMR $\delta^{TMS}_{CDCl_3}$ 0.80 (m, terminal methyl), 1.20 (s, gem dimethyl), 2.9 (m), 3.0—3.6 (m), 4.30 (s, benzylic methylene), 4.5—5.1 (m), 5.00 (s, benzylic methylene), 6.78 (m, ArH), 6.98 (d, J=8Hz, ArH), 7.17 (s, PhH) and 7.30 (s, PhH).

Diastereomer B:

m.p.: 65—69°C.

$[\alpha]_D^{25°}$ = +40.95° (C = 1.21, $CH_3OH$).

PMR $\delta^{TMS}_{CDCl_3}$ 0.80 (m, terminal methyl), 1.20 (s, gem dimethyl), 2.8 (m), 3.0—3.5 (m), 4.26 (s, benzylic methylene), 4.4—5.1 (m), 4.95 (s, benzylic methylene), 6.70 (m, ArH), 6.87 (d, J=8Hz), 7.12 (s, PhH) and 7.20 (s, PhH).

Example 19

Enantiomer A of *Cis*-3-[2-Benzyloxy-4-(1,1-dimethylheptyl)-phenyl]-*trans*-4-(3-hydroxypropyl)cyclohexanol

A mixture of 18 g (24.5 mmole) of diastereomer A of *cis*-3-[2-benzyloxy-4-(1,1-dimethylheptyl)phenyl]-*trans*-4-(3-hydroxypropyl)cyclohexanol bis-*d*-mandelate and 13.5 g (98.1 mmole) of potassium carbonate in 250 ml methanol, 175 ml tetrahydrofuran and 35 ml water was stirred for 24 hours. The reaction was added to one liter saturated sodium chloride and extracted twice with 500 ml portions of ether. The combined organic extract was dried over magnesium sulfate and evaporated to a quantitative yield of the title compound as an oil.

$[\alpha]_D^{25°} = -34.51°$ (C = 1.083, methanol).

PMR $\delta_{CDCl_3}^{TMS}$ 0.82 (m, terminal methanol), 1.25 (s, gem dimethyl), 2.90 (m, benzylic methine), 3.40 (bt, $CH_2OH$), 3.7 (m, carbinol methine), 5.06 (s, benzylic methylene), 6.82 (d, J=2Hz, ArH), 6.82 (dd, J=8 and 2Hz, ArH), 7.00 (d, J=8Hz, ArH) and 7.32 (s, PhH).

Using the above procedure, 4.00 g (5.79 mmole) of *cis*-3-[2-benzyloxy-4-(1,1-dimethylheptyl)phenyl]-*trans*-4-(3-benzyloxypropyl)-1-*d*-mandeloyloxycyclohexane, diastereomer A, gave a quantitative yield of *cis*-3-[2-benzyloxy-4-(1,1-dimethylheptyl)phenyl]-*trans*-4-(3-benzyloxypropyl)cyclohexanol, enantiomer A, as an oil.

$[\alpha]_D^{25°} = -24.92°$ (C = 1.23, $CH_3OH$).

PMR $\delta_{CDCl_3}^{TMS}$ 0.82 (m, terminal methyl), 1.22 (s, gem dimethyl), 2.9 (m, benzylic methine), 3.23 (bt, J=6Hz, methylene), 3.7 (m, carbinol methine), 4.35 and 5.00 (s, benzylic methylenes), 6.82 (m, ArH), 7.02 (d, J=8Hz, ArH), 7.19 and 7.35 (s, PhH).

Example 20

Enantiomer B of *Cis*-3-[2-Benzyloxy-4-(1,1-dimethylheptyl)phenyl]-*trans*-4-(3-hydroxypropyl)cyclohexanol

a. Using the procedure of Example 19, 21 g (28.6 mmole) of diastereomer B of *cis*--3-[2-benzyloxy-4-(1,1-dimethylheptyl)phenyl]-*trans*-4-(3-hydroxypropyl)cyclohexanol bis-*d*-mandelate gave 13 g (98%) of the title compound as an oil.

$[\alpha]_D^{25°} = +26.30°$ (C = 1.051, $CH_3OH$).

b. Using the procedure of Example 18, 13 g (27.8 mmole) of the title compound and 10.6 g (69.7 mmole) of *l*-mandelic acid gave 8 g (39%) of diastereomer A of *cis*-3-[2-benzyloxy-4-(1,1-dimethylheptyl)phenyl]-*trans*-4-(3-hydroxypropyl)cyclohexanol bis-*l*-mandelate.

m.p. 106—107.5°C (methanol).

PMR $\delta_{CDCl_3}^{TMS}$ 0.83 (terminal methyl), 1.25 (s, gem dimethyl, 2.85 (m, benzylic methine), 3.40 (m), 4.0 (m), 4.7—5.1 (m), 5.07 (s, benzylic methylene).

| | | |
|---|---|---|
| *Analysis:* Calc'd for $C_{47}H_{58}O_7$: | C, 76.81; | 7.95. |
| *Found:* | C, 76.69; | H, 7.86. |

c. Using the procedure of Example 19, 8.0 g (10.9 mmole) of diastereomer A of *cis*-3-[2-benzyloxy-4-(1,1-dimethylheptyl)phenyl]-*trans*-4-(3-hydroxypropyl)cyclohexanol bis-*l*-mandelate gives 4.7 g (93%) of the title compound.

$[\alpha]_D^{25°} = +34.17°$ (C = 1.15, $CH_3OH$).

PMR $\delta_{CDCl_3}^{TMS}$ 0.86 (m, terminal methyl), 1.28 (s, gem dimethyl), 2.82 (m, benzylic methine), 3.42 (bt, $CH_2OH$), 3.7 (m, carbinol methine), 5.10 (s, benzylic methylene), 6.9 (m, ArH), 7.10 (d, J=8Hz, ArH) and 7.40 (s, PhH).

Example 21

*Cis*-3-[4-(1,1-Dimethylheptyl)-2-hydroxyphenyl]-*trans*-4-(3-hydroxypropyl)cyclohexanol, Enantiomer A

Using the procedure of Example 5, 11.4 g (24.5 mmole) of enantiomer A of *cis*-3-[2-benzyloxy-4-(1,1-dimethylheptyl)phenyl]-*trans*-4-(3-hydroxypropyl)cyclohexanol gave 8.5 g (93%) of the title compound.

m.p. 65—72°C (hexane-dichloromethane).

$[\alpha]_D^{25°} = -39.24°$ (C = 1.00, $CH_3OH$).

HRMS (m/e) 376.2938 ($M^+$, calc'd for $C_{24}H_{40}O_3$: 376.2967), 304, 273, 255, 199, 147 and 121.

PMR $\delta_{CDCl_3}^{TMS}$ 0.83 (m, terminal methyl), 1.23 (s, gem dimethyl), 2.83 (m, benzylic methine), 3.58 (bt, $CH_2OH$), 3.7 (carbinol methine), 6.9 (m, ArH) and 7.00 (d, J=8Hz, ArH).

Example 22

*Cis*-3-[4-(1,1-Dimethylheptyl)-2-hydroxyphenyl]-*trans*-4-(3-hydroxypropyl)cyclohexanol, Enantiomer B

Using the procedure of Example 5, 4.7 g (10.1 mmole) of enantiomer B of *cis*-3-[2-benzyloxy-4-(1,1-dimethylheptyl)phenyl]-*trans*-4-(3-hydroxypropyl)cyclohexanol gave 3.5 g (92%) of the title compound as an oil.

$[\alpha]_D^{25°} = +36.47°$ (C = 0.947, $CH_3OH$).

HRMS (m/e) 376.2942 ($M^+$, calc'd for $C_{24}H_{40}O_3$: 376.2967), 358, 291, 273 and 147.

PMR ($CDCl_3$) δ 0.82 (s, terminal methyl), 1.2 (s, gem dimethyl) 2.7 (m, benzylic methine), 3.41 (bt, J=7Hz, carbinol methylene), 3.78 (m, carbinol methine), 6.77 (m, ArH) and 7.00 (d, J=8Hz, ArH).

Example 23

*Trans*-4-(2-Propenyl)-3-[2-(tetrahydropyranyl-2-oxy)-4-(1,1-dimethyl)-*cis*-2-heptenyl)phenyl]cyclohexanone

To a 0°C solution of 2.0 g (6.62 mmole) of 1,1-dimethyl-1-[3-(tetrahydropyranyl-2-oxy)phenyl]-*cis*-2-heptene and 844 mg (7.28 mmole) of tetramethylethylene diamine in 10 ml of ether was slowly added 3.17 ml (7.28 mmole) of m-butyllithium (in hexane). The resultant solution was stirred 5 minutes at 0°C and then refluxed for 70 minutes. The resultant was then cooled to −78°C.

To a 0°C solution of 597 mg (7.28 mmole) of 1-hexyne in 10 ml of tetrahydrofuran is slowly added 3.17 ml (7.28 mmole) of *m*-butyllithium (in hexane). The resultant solution was stirred 10 minutes at 0°C and then slowly added to a 0°C slurry of 1.38 g (7.28 mmole) of cuprous iodide in 10 ml of tetrahydrofuran. The resultant yellow slurry was stirred 30 minutes at 0°C and then slowly added to the above prepared −78°C solution of aryl lithium reagent. The resultant yellow mixture was stirred 10 minutes at −78°C and then 900 mg (6.62 mmole) of 4-(2-propenyl)cyclohex-2-en-1-one is added dropwise. Stirring was continued for 10 minutes and then the reaction was placed in a −40°C cooling bath and allowed to warm to −20°C over 20 minutes. The reaction was quenched by addition to 200 ml of cold saturated ammonium chloride brought to pH 10 with saturated ammonium hydroxide. The quenched reaction mixture was extracted with 300 ml of ether. The ether extract was dried over magnesium sulfate and evaporated to an oil which was purified via column chromatography on 200 g of silica gel eluted in 15 ml fractions with 20% etherhexane to yield from fractions 40—63, 2.36 g (82%) of the title compound as an oil.

IR ($CHCl_3$) 1709, 1641, 1610 and 1571 $cm^{-1}$.

HRMS (m/e) 354.2569 ($P^+$. —$C_5H_8O$: Calcd. for $C_{24}H_{32}O_2$: 354.2550), 272, 271, 161, 137 and 85 (100%).

PMR ($CDCl_3$) δ 0.69 (m, terminal $CH_3$), 1.41 (s, gem dimethyl), 0.8—4.2 (series of m), 4.7—5.6 (m, olefinic H and THP methine), 5.62 (d, J=12Hz, vinyl H) and 6.7—7.3 (m, ArH).

Example 24

*Trans*-4-(2-Propenyl)-cis-3-[2-(tetrahydropyranyl-2-oxy)-4-(1,1-dimethyl-*cis*-2-heptenyl)phenyl]cyclohexanol and the *cis*-4,trans-3 isomer

To a −78°C solution of 20.0 g (45.7 mmole) of *trans*-4-(2-propenyl)-3-[2-(tetrahydropyranyl-2-oxy)-4-(1,1-dimethyl-*cis*-2-heptenyl)phenyl]cyclohexanone in 200 ml of methanol was added in one portion 5.20 g (137 mmole) of sodium borohydride. The reaction was stirred overnight at −78°C and then allowed to warm to 0°C. The reaction was quenched with 10 ml saturated sodium chloride and then concentrated on a rotovapor. The residue was diluted with 500 ml saturated sodium chloride and 500 ml ether. The ether extract was washed once with 250 ml saturated sodium bicarbonate, dried over magnesium sulfate and evaporated to an oil. The crude oil was purified via column chromatography on 1 kg of silica gel eluted with 30% ether-hexane to yield in order of elution 2.20 g (11%) of the *cis*-4,*trans*-3 isomer, 0.74 g (4%) of mixture and 17.0 g (85%) of the title compound as oils.

Title Compound:

IR ($CHCl_3$) 3593, 3464, 1640, 1610 and 1571 $cm^{-1}$.

HRMS (m/e) 356.2709 ($P^+$. —$C_5H_8O$, Calcd. for $C_{24}H_{36}O_2$: 356.2706), 297, 273, 255, 124 and 85 (100%).

PMR ($CDCl_3$) δ 0.70 (m, terminal $CH_3$), 1.42 (s, gem dimethyl), 2.8 (bm, benzylic methine), 3.4—4.1 (m, carbinol methine and $OCH_2$), 4.6—5.5 (m, vinyl H and THP methine), 5.60; (d, J=11Hz, vinyl H) and 6.8—7.2 (m, ArH).

Example 25

*Trans*-4-(3-Hydroxypropyl)-*cis*-3-[2-(tetrahydropyranyl-2-oxy)-4-(1,1-dimethyl-*cis*-2-heptenyl)phenyl]cyclohexanol

To a −5°C. solution of 13.3 g. (0.0302 mole) of *trans*-4-(2-propenyl)-*cis*-3-[2-(tetrahydropyranyl-2-oxy)-4-(1,1-dimethyl-*cis*-2-heptenyl)phenyl]cyclohexanol in 100 ml. of tetrahydrofuran was slowly added 35.1 ml. (0.0351 mole) of boran-tetrahydrofuran (1 M in tetrahydrofuran), with stirring. After 30 minutes another 50 ml. of tetrahydrofuran was added and the reaction stirred 30 minutes longer, then quenched by addition of 40 ml. (0.12 mole) of 3N sodium hydroxide and oxidized by addition of 10 ml. (0.112 mole) of 30% hydrogen peroxide. After stirring 20 minutes the reaction was added to 500 ml. saturated sodium chloride — 500 ml. ether. The aqueous phase was extracted with another 250 ml. portion of ether and the combined ether extract dried over magnesium sulfate and evaporated to an oil. The crude oil was purified via column chromatography on 1 kg. of silica gel eluted with 80% ether-hexane to 3% methanol-ether to yield in order of elution 600 mg. (5%) of unreacted starting material, 7.45 g. (54%) of the title compound as an oil, 1.32 g. (12%) of *trans*-4-(3-hydroxypropyl)-*cis*-3-[2-hydroxy-4-(1,1-dimethyl-*cis*-2-heptenyl)phenyl]cyclohexanol (phenolic product) and 1.78 g. (13%) of *trans*-4-(3-hydroxypropyl)-*cis*-3-[2-(tetrahydropyranyl-2-oxy)-4-(1,1-dimethyl-3-hydroxyheptyl)phenyl]cyclohexanol (Triol-THP).

Title Compound:

HRMS (m/e) 374.2831 ($P^+$ —$C_5H_8O$, Calcd. for $C_{24}H_{38}O_3$: 374.2811), 356, 242, 151 and 85.

PMR ($CDCl_3$) δ 0.72 (m, terminal $CH_3$), 1.40 (s, gem dimethyl), 2.8 (m, benzylic methine), 3.47 (m, $\underline{CH_2}OH$), 3.2—4.2 (m, carbinol methine and —$CH_2O$—), 4.9—5.5 (vinyl H and THP methine), 5.62 (d, J=11 Hz, vinyl H) and 6.9—7.2 (m, ArH).

Phenotic Product:

HRMS (m/e) 374.2839 ($M^{+}$, Calcd. for $C_{24}H_{38}O_3$: 374.2811), 356, 341, 299, 273 and 270.

Triol-THP:

HRMS (m/e) 392.2949 ($P^{+}$ —$C_5H_8O$, Calcd. for $C_{24}H_{40}O_4$: 392.2916), 374, 292, 274, 165 and 85 (100%).

Example 26

*Trans*-4-(3-*d*-Mandeloyloxy)-*cis*-3-[2-hydroxy-4-(1,1-dimethyl-*cis*-2-heptenyl)phenyl]-1-*d*-mandeloyloxycyclohexane

A mixture of 6.00 g. (13.1 mmole) of *trans*-4-(3-hydroxypropyl)-*cis*-3-[2-(tetrahydropyranyl-2-oxy)-4-(1,1-dimethyl-*cis*-2-heptenyl)phenyl]cyclohexanol, 9.96 g. (65.5 mmole) *d*-mandelic acid and 498 mg. (2.62 mmole) *p*-toluenesulfonic acid monohydrate in 250 ml. of benzene was heated at reflux. Water was removed via a 3A molecular sieve filled soxhlet extractor. The reaction was heated at reflux for 160 minutes with additional portions of *p*-toluenesulfonic acid monohydrate added at 40, 80 and 120 minutes. The reaction was cooled and added to 500 ml. saturated sodium bicarbonate — 500 ml. ether. The aqueous phase was extracted with a second 250 ml. portion of ether and the combined ether extract dried over magnesium sulfate and evaporated to an oil. The crude product was purified via column chromatography on 800 g. of silica gel eluted with 55% ether-hexane to yield order of elution 2.25 g. (27%) of diastereomer A of the title compound, 2.0 g. (24%) of a mixture and 4.0 g. (48%) of slightly impure diastereomer B of the title compound as oils.

Diastereomer A:

$20°[\alpha]_D^{CH_3OH} = +31.62°$ (C=1.85)

PMR ($CDCl_3$) δ 0.68 (m, terminal $CH_3$), 1.37 (s, gem dimethyl), 3.4 (bd, J=6 Hz, OH), 3.93 (bt, J=6 Hz, —$CH_2O$—), 4.75 (m, OH), 5.02 (m, <u>CH</u>OH), 5.0—5.7 (m, vinyl H), 6.6—6.9 (m, ArH), 7.23 and 7.26 (s, PhH).

Example 27

(−)-*Trans*-4-(3-hydroxypropyl)-*cis*-3-[2-hydroxy-4-(1,1-dimethyl-*cis*-2-heptenyl)phenyl]cyclohexanol

A mixture of 3.10 g. (4.82 mmole) of diastereomer A of *trans*-4-(3-*d*-mandeloyloxy)-*cis*-3-[2-hydroxy-4-(1,1-dimethyl-*cis*-2-heptenyl)phenyl]-1-*d*-mandeloyloxycyclohexane, 5.32 g. (38.6 mmole) potassium carbonate, 40 ml. methanol, 40 ml. tetrahydrofuran and 10 ml. water was stirred at 25°C. for 24 hours. The reaction was evaporated on a rotovapor and the residue dissolved in 250 ml. ether — 250 ml. saturated sodium chloride. The aqueous phase was extracted with two additional 150 ml. portions of ether, the combined ether extract dried over magnesium sulfate and evaporated to an oil. The crude product was purified via column chromatography on 150 g. of silica gel eluted with ether to yield 1.3 g. (72%) of the title compound.

MP: 95—97°C. (Diisopropyl ether-hexane)

$20°[\alpha]_D^{CH_3OH} = -56.32°$

HRMS (m/e) 374.2819 ($M^{+}$, Calcd. for $C_{24}H_{38}O_3$: 374.2811), 356, 213, 187, 161, 147, 124, 121, and 93.

PMR (250 mHz, $CDCl_3$) δ 0.73 (m, terminal methyl), 1.39 (s, gem dimethyl), 2.03 (m, $CH_2$-vinylic), 2.72 (m, benzylic methine), 3.47 (m, <u>$CH_2$</u>OH), 3.74 (m, <u>CH</u>OH), 5.07 (bs, OH), 5.22 and 5.27 (dt, J=11.51 and 7.31 Hz, vinyl H), 5.61 (dt, J=11.51 and 1.6 Hz, vinyl H), 6.73 (d, J=1.64 Hz, ArH), 6.91 (dd, J=8.22 and 1.82 Hz, ArH) and 7.02 (d, J=8.04 Hz, ArH).

Example 28

(−)-*Trans*-4-(3-hydroxypropyl)-*cis*-3-[2-hydroxy-4-(1,1-dimethylheptyl)phenyl]cyclohexanol

A mixture of 50.0 mg. (0.134 mmole) of (−)-*trans*-4-(3-hydroxypropyl)-*cis*-3-[2-hydroxy-4-(1,1-dimethyl-*cis*-2-heptenyl)phenyl]cyclohexanol and 50 mg. of 10% palladium on carbon in 5 ml. of dry tetrahydrofuran was stirred under one atmosphere of hydrogen for 30 minutes. The reaction was filtered through diatomaceous earth with tetrahydrofuran and the filtrate evaporated to yield 47.9 mg. (95%) of the title compound as an oil.

$20°[\alpha]_D^{CH_3OH} = -36.12°$.

Example 29

Trans-3,4,*trans*-4,5-3-[2-benzyloxy-4-(1,1-dimethylheptyl)phenyl]-5-methyl-4-(2-propenyl)cyclohexanone

Using the procedure of Example 1, 7.5 g. (50 mmole) of *trans*-5-methyl-4-(2-propenyl)cyclohex-2-en-1-one and 24.3 g. (62.5 mmole) of 1-benzyloxy-2-bromo-5-(1,1-dimethylheptyl)benzene gave 13.0 g. (57%) of the title compound as an oil.

IR ($CHCl_3$) 1709, 1639, 1609 and 1568 $cm^{-1}$.

HRMS (m/e) 460.3353 ($M^{+}$, Calcd. for $C_{32}H_{44}O_2$: 460.3330), 375, 370, 369 and 91 (100%).

PMR ($CDCl_3$) δ 0.82 (m, terminal $CH_3$), 1.26 (s, gem, dimethyl), 4.5—5.1 (m, vinyl H), 5.00 (s, benzylmethylene), 5.3—5.8 (vinyl H), 6.6—7.1 (m, ArH) and 7.25 (bs, PhH).

In like manner, *trans*-3,4-*trans*-4,5-3-[2-benzyloxy-4-(1,1-dimethylheptyl)phenyl]-5-methyl-4-(2-propenyl)cycloheptanone is prepared from *trans*-5-methyl-4-(2-propenyl)cyclohept-2-en-1-one.

Example 30

*Cis*-3-[2-benzyloxy-4-(1,1-dimethylheptyl)phenyl]-*cis*-5-methyl-trans-4-(2-propenyl)cyclohexanol

Using the procedure of Example 2, 6.0 g. (13.0 mmole) of trans-3,4-*trans*, 4,5-3-[2-benzyloxy-4-(1,1-dimethylheptyl)phenyl]-5-methyl-4-(2-propenyl)cyclohexanone gave 1.1 g. (18%) of the *cis*-4,*trans*-3,*trans*-5 isomer of the title compound, 1.077 g. (17%) of a mixture and 2.79 (46%) of the title compound as an oil.

*Cis*-4,*Trans*-3,*Trans*-5 Isomer:

HRMS (m/e) 462.3501 ($M^{+}$, Calcd. for $C_{32}H_{46}O_2$: 362.3486), 377, 269, 227 and 91 (100%).

PMR ($CDCl_3$) δ 0.82 (m, terminal methyl), 0.95 (d, J=6 Hz, methyl), 1.23 (s, gem dimethyl), 3.5 (m, benzylic methine), 4.13 (m, carbinol methine), 4.6—5.0 (m, vinyl H), 5.05 (s, benzylic methylene), 5.2—6.1 (m, vinyl H), 6.85 (m, ArH), 7.05 (d, J=8 Hz, ArH) and 7.38 (bs, PhH).

Title Compound:

IR ($CHCl_3$) 3596, 3463, 1639, 1609 and 1570 $cm^{-1}$.

HRMS (m/e) 462.3499 ($M^{+}$, Calcd. for $C_{32}H_{46}O_2$: 462.3501), 377, 353, 269, 227 and 91 (100%).

PMR ($CDCl_3$) δ 0.83, (m, terminal $CH_3$), 1.25 (s, gem dimethyl), 3.2 (m, benzylic methine), 3.62 (bm, carbinol methine), 4.5—5.0 (m, vinyl H), 5.02 (s, benzylic methylene), 5.3—6.1 (m, vinyl H), 6.82 (m, ArH), 7.00 (d, J=8 Hz, ArH) and 7.35 (bs, PhH).

Following the above procedure *trans*-3,4-*trans*-4,5-3-[2-benzyloxy-4-(1,1-dimethylheptyl)phenyl]-5-methyl-*trans*-4-(2-propenyl)cycloheptanone is prepared from the correspondingly substituted cyclohept-2-en-1-one.

Example 31

*Trans*-3,4-*Trans*,4,5-3-[2-benzyloxy-4-(1,1-dimethylheptyl)phenyl]-5-methyl-4-(2-propenyl)cyclohexanone ethylene ketal

A mixture of 7.0 g. (15.2 mmole) of trans-3,4-*trans*,4,5-3-[2-benzyloxy-4-(1,1-dimethylheptyl)phenyl]-5-methyl-4-(2-propenyl)cyclohexanone, 8.47 ml. (152 mmole) of ethylene glycol and 289 mg. (1.52 mmole) of *p*-toluenesulfonic acid monohydrate was heated at reflux for 2.5 hours. Water was removed via a soxhlet extractor filled with 3A sieves. The reaction was cooled and added to 250 ml. saturated sodium bicarbonate — 250 ml. ether. The ether extract was dried over magnesium sulfate and evaporated to an oil. The crude product was purified via column chromatography on 300 g. of silica gel eluted with 20% ether-hexane to yield 5.2 g. (68%) of the title compound as an oil.

IR ($CHCl_3$) 1638, 1608 and 1569 $cm^{-1}$.

HRMS (m/e) 504.3579 ($M^{+}$, Calcd. for $C_{34}H_{48}O_3$: 504.3591), 419, 413, 407 and 91 (100%).

PMR ($CDCl_3$) δ 0.82 (m, terminal methyl), 0.99 (d, J=6 Hz, $CH_3$), 1.25 (s, gem dimethyl), 3.4 (m, benzylic methine), 3.90 (s, ethylene ketal), 4.4—5.0 (m, vinyl H), 5.03 (s, benzylic methylene), 5.3—6.2 (m, vinyl H), 6.80 (m, ArH), 7.00 (d, J=8 Hz, ArH) and 7.35 (bs, PhH).

The ethylene ketal of *trans*-3,4-*trans*-4,5-3-[2-benzyloxy-4-(1,1-dimethylheptyl)phenyl]-5-methyl-4-(2-propenyl)cycloheptanone is prepared in like manner from the corresponding cycloheptanone.

Example 32

*Cis*-3-[4-(1,1-dimethylheptyl)-2-hydroxyphenyl]-*cis*-5-methyl-4-(2-propenyl)cyclohexanol

To a 0°C. solution of 900 mg. (1.95 mmole) of *cis*-3-[2-benzyloxy-4-(1,1-dimethylheptyl)phenyl]-*cis*-5-methyl-*trans*-4-(2-propenyl)cyclohexanol in 2 ml. of ether was added 5.1 ml. (11.7 mmole) of *n*-butyllithium (in hexane). The reaction was stirred 10 hours at 25°C. and then added to 200 ml. ether — 200 ml. saturated ammonium chloride. The ether extract was dried over magnesium sulfate and evaporated to an oil. This oil was crystallized in hexane to yield 495 mg. (68%) of the title compound.

MP 149—150°C. (hexane)

IR ($CHCl_3$) 3455, 3253, 1643, 1617 and 1583 $cm^{-1}$.

HRMS (m/e) 372.3050 ($M^{+}$, Calcd. for $C_{25}H_{40}O_2$: 372.3018), 312, 288, 287 (100%), 272, 269, 227, 187, 161, 147 and 135.

*Analysis:*

| | | |
|---|---|---|
| Analysis Calcd. for $C_{25}H_{40}O_2$: | C, 80.59; | H, 10.82 |
| Found: | C, 80.35; | H, 10.81 |

Debenzylation of *cis*-3-[2-benzyloxy-4-(1,1-dimethylheptyl)phenyl]-*cis*-5-methyl-*trans*-4-(2-propenyl)cycloheptanol is carried out in like manner.

Example 33

*Cis*-3-[2-Benzyloxy-4-(1,1-dimethylheptyl)phenyl]-*trans*-4-(3-hydroxypropyl)-*cis*-5-methylcyclohexanol

Using the procedure of Example 4, 900 mg. (1.95 mmole) of *cis*-3-[2-benzyloxy-4-(1,1-dimethylheptyl)phenyl]-*cis*-5-methyl-*trans*-4-(2-propenyl)cyclohexanol was converted in quantitative yield to the title compound, an oil.

HRMS (m/e) 480.3574 ($M^{+}$, Calcd. for $C_{32}H_{48}O_3$: 480.3591), 462, 395, 377, 287 and 91 (100%).

By means of this procedure, *cis*-3-[2-benzyloxy-4-(1,1-dimethylheptyl)phenyl]-*cis*-5-methyl-*trans*-4-(2-propenyl)cycloheptanol is converted to the corresponding 3-hydroxypropyl derivative.

Example 34

*Cis*-3-[4-(1,1-dimethylheptyl)-2-hydroxyphenyl]-*trans*-4-(3-hydroxypropyl)-*cis*-5-methylcyclohexanol

Using the procedure of Example 5, 936 mg. (1.95 mmole) of *cis*-3-[2-benzyloxy-4-(1,1-dimethylheptyl)phenyl]-*trans*-4-(3-hydroxypropyl)-*cis*-5-methylcyclohexanol afforded 602 mg. (79%) of the title compound.

MP 159—160°C. (diisopropyl ether — ethyl acetate)

IR (KBr) 3533, 3367, 3211, 1617 and 1585 $cm^{-1}$.

HRMS (m/e) 390.3100 ($M^{+}$, Calcd. for $C_{25}H_{42}O_3$, 390.3123), 372, 304, 288, 287, 272, 257, 227, 219 and 187.

*Analysis:*

| | | |
|---|---|---|
| Anal. Calcd. for $C_{25}H_{42}O_3$: | C, 76.87; | H, 10.84. |
| Found: | C, 76.60; | H, 10.66. |

Similarly, *cis*-3-[2-benzyloxy-4-(1,1-dimethylheptyl)phenyl]-*trans*-4-(3-hydroxypropyl)-*cis*-5-methylcycloheptanol is debenzylated to the corresponding phenol.

Example 35

*Trans*-3,4,*Trans*-4,5-3-[2-benzyloxy-4-(1,1-dimethylheptyl)phenyl]-4-(2-hydroxypropyl)-5-methylcyclohexanone ethylene ketal

To a 25°C. mixture of 3.28 g. (10.3 mmole) of mercuric acetate in 50 ml. of 50% aqueous tetrahydrofuran was added 5.2 g. (10.3 mmole) of *trans*-3,4,*trans*-4,5-3-[-2-benzyloxy-4-(1,1-dimethylheptyl)phenyl]-5-methyl-4-(2-propenyl)cyclohexanone ethylene ketal in 25 ml. tetrahydrofuran. Another 25 ml. of water was added to aid solution and stirring. Additional 1.5 g. (4.7 mmole) portions of mercuric acetate were added after 2 hours and 24 hours. The reaction was stirred a total of 64 hours at 25°C. and then reduced by addition of 20 ml. 3N sodium hydroxide and 20 ml. of 0.5 M sodium borohydride in 3N sodium hydroxide. The reaction was stirred 30 minutes longer and then added to 250 ml. ether — 300 ml. saturated sodium chloride. The ether extract was dried over magnesium sulfate and evaporated to an oil. The crude product was purified via column chromatography on 300 g. of silica gel eluted with 50% ether-hexane to give 1.5 g. (30%) of the title compound as an oil.

HRMS (m/e) 522.3712 ($M^{+}$, Calcd. for $C_{34}H_{50}O_4$: 522.3696), 431, 407, 372, 113 and 91 (100%).

PMR ($CDCl_3$) δ 0.75 (m, terminal methyl), 0.92 (d, J=6 Hz, C—5 methyl), 1.24 (s, gem dimethyl), 3.35 (bm, carbinol and benzylic methines), 3.90 (bs, ethylene ketal), 5.03 (s, benzylic methylene) and 6.6—7.5 (m, ArH).

*Trans*-3,4-*trans*-4,5-3-[2-benzyloxy-4-(1,1-dimethylheptyl)phenyl]-4-(2-hydroxypropyl)-5-methylcycloheptanone ethylene ketal is also prepared via this procedure from the corresponding 4-(2-propenyl)cycloheptanone ethylene ketal.

Example 36

*Trans*-3,4,*Trans*-4,5-3-[2-Benzyloxy-4-(1,1-dimethylheptyl)phenyl]-5-methyl-4-(2-oxopropyl)cyclohexanone ethylene ketal

To a 25°C. mixture of 1.5 g. (2.87 mmole) of *trans*-3,4,*trans*-4,5-3-[2-benzyloxy-4-(1,1-dimethylheptyl)phenyl]-4-(2-hydroxypropyl)-5-methylcyclohexanone ethylene ketal and 2.35 g. (28.7 mmole) of sodium acetate in 20 ml. of dichloromethane was added 1.93 g. (9 mmole) of pyridinium chlorochromate. The reaction mixture was stirred 3 hours at 25°C. and then added to 250 ml. 1N NaOH — 250 ml. ether. The organic extract was dried over magnesium sulfate and evaporated to give a quantitative yield of the title compound as an oil.

PMR ($CDCl_3$) δ 0.85 (m, methyls), 1.23 (s, gem dimethyl), 1.80 (bs, $CH_3CO$—), 2.23 (bs, —$CH_2CO$—), 3.3 (m, benzylic methine), 3.93 (s, ethylene ketal), 5.05 (s, benzylic methylene) and 6.7—7.6 (m, ArH).

In like manner, oxidation of *trans*-3,4-*trans*-4,5-3-[2-benzyloxy-4-(1,1-dimethylheptyl)phenyl]-4-(2-hydroxypropyl)cycloheptanone ethylene ketal affords the corresponding 4-(2-oxopropyl)cycloheptanone ethylene ketal.

Example 37

*Trans*-3,4-*Trans*-4,5-3-[2-Benzyloxy-4-(1,1-dimethylheptyl)phenyl]-5-methyl-4-(2-methyl-2-propenyl)cyclohexanone ethylene ketal

To a 15°C. mixture of 5.74 mmole of triphenyl phosphoniummethylide in 6 ml. of dimethyl sulfoxide was added a solution of 1.5 g. (2.87 mmole) of *trans*-3,4-*trans*-4,5-3-[2-benzyloxy-4-(1,1-dimethylheptyl)phenyl]-5-methyl-4-(2-oxopropyl)cyclohexanone ethylene ketal in 6 ml. of tetrahydrofuran and the reaction stirred 1.5 hours at 15—25°C. It was then added to 200 ml. saturated sodium chloride — 200 ml. ether. The ether extract was washed once with 200 ml. saturated sodium chloride, dried over magnesium sulfate and evaporated to give an oil. Purification via column chromatography on 50 g. of silica gel eluted with 20% ether-hexane gave 1.17 g. (79%) of the title compound as an oil.

HRMS (m/e) 518.3706 (M⁺̇, Calcd. for $C_{35}H_{50}O_3$: 518.3747), 462, 427, 407, 372, 371, 285 and 91 (100%).

PMR ($CDCl_3$) δ 1.25 (s, gem dimethyl), 1.48 (bs, vinyl methyl), 3.3 (m, benzylic methine), 3.89 (s, ethylene ketal), 4.40 (m, vinyl H), 5.02 (s, benzylic methylene), 6.85 (m, ArH), 7.10 (d, J=8 Hz, ArH) and 7.4 (m, PhH).

*Trans*-3,4-*trans*-4,5-3-[2-benzyloxy-4-(1,1-dimethylheptyl)phenyl]-5-methyl-4-(2-oxopropyl)cycloheptanone ethylene ketal is converted in like manner to the corresponding 4-(2-methyl-2-propenyl)cycloheptanone ethylene ketal.

Example 38

*Trans*-3,4-*trans*-4,5-3-[2-Benzyloxy-4-(1,1-dimethylheptyl)phenyl]-5-methyl-4-(2-methyl-2-propenyl)cyclohexanone

A mixture of 1.1 g. (2.12 mmole) of the ethylene ketal of the title compound, 20 ml. tetrahydrofuran and 20 ml. 1N hydrochloric acid was heated at reflux for 5 hours and stirred at 25°C. for 16 hours. The reaction was added to 250 ml. saturated sodium chloride — 250 ml. ether. The ether extract was washed with 250 ml. saturated sodium bicarbonate, dried over magnesium sulfate and evaporated to give a quantitative yield of the title compound as an oil.

HRMS (m/e) 474.3524 (M⁺̇, Calcd. for $C_{33}H_{46}O_2$): 474.3486), 418, 383, 327, 273 and 91 (100%).

Deketalization of *trans*-3,4-*trans*-4,5-3-[2-benzyloxy-4-(1,1-dimethylheptyl)phenyl]-5-methyl-4-(2-methyl-2-propenyl)cycloheptanone ethylene ketal is accomplished in like manner.

Example 39

*Cis*-3-[2-Benzyloxy-4-(1,1-dimethylheptyl)phenyl]-*cis*-5-methyl-*trans*-4-(2-methyl-2-propenyl)cyclohexanol and its axial Isomer

Using the procedure of Example 9, 1.00 g. (2.12 mmole) of *trans*-3,4-*trans*-4,5-3-[2-benzyloxy-4-(1,1-dimethylheptyl)phenyl]-*cis*-5-methyl-*trans*-4-(2-methyl-2-propenyl)cyclohexanone gave 248 mg. (25%) of the *trans*-3,*cis*-4,*trans*-5 isomer of the title compound and 720 mg. (71%) of the title compound as oils.

*Trans*-3,*cis*-4,*trans*-5 Isomer:

HRMS (m/e) 476.3664 (M⁺̇, Calcd. for $C_{33}H_{48}O_2$: 476.3642), 420, 335, 330, 329 (100%), 312, 311, and 283.

Title Compound:

HRMS (m/e) 476.3646 (M⁺̇, Calcd. for $C_{33}H_{48}O_2$: 476.3642), 420, 330, 329 and 91 (100%).

Similarly *trans*-3,4-*trans*-4,5-3-[2-benzyloxy-4-(1,1-dimethylheptyl)phenyl]-*cis*-5-methyl-*trans*-4-(2-methyl-2-propenyl)cycloheptanone is transformed to *cis*-3-[2-benzyloxy-4-(1,1-dimethylheptyl)phenyl]-*cis*-5-methyl-*trans*-4-(2-methyl-2-propenyl)cycloheptanol and the corresponding axial derivative.

Example 40

*Cis*-3-[2-Benzyloxy-4-(1,1-dimethylheptyl)phenyl]-*trans*-4-(3-hydroxy-2-methylpropyl)-*cis*-5-methylcyclohexanol

Using the procedure of Example 7, 710 mg. (1.49 mmole) of *cis*-3-[2-benzyloxy-4-(1,1-dimethylheptyl)phenyl]-*cis*-5-methyl-*trans*-4-(2-methyl-2-propenyl)cyclohexanol (compound of Example 39) gave 322 mg. (44%) of diastereomer A and 356 mg. (48%) of diastereomer B of title compound as oils.

Diastereomer A:

HRMS (m/e) 494.3769 (M⁺̇, Calcd. for $C_{33}H_{50}O_3$: 494.3747), 386, 301 and 91 (100%).

Diastereomer B:

HRMS (m/e) 494.3790 (M⁺̇, Calcd. for $C_{33}H_{50}O_3$: 494.3747) and 91 (100%).

In like manner, the remaining alkenyl derivatives of Example 39 are hydrated to the corresponding alcohols.

Example 41

*Cis*-3-[4-(1,1-dimethylheptyl)-2-hydroxyphenyl]-*trans*-4-(3-hydroxy-2-methylpropyl)-*cis*-5-methylcyclohexanol

Diastereomer A

Using the procedure of Example 5, 320 mg. (0.648 mmole) of diastereomer A of *cis*-3-[2-benzyloxy-4-(1,1-dimethylheptyl)phenyl]-*trans*-4-(3-hydroxy-2-methylpropyl)-*cis*-5-methylcyclohexanol (product of Example 40) provided 221 mg. (84%) of the title compound.

MP 139—141°C. (diisopropyl ether-hexane)

IR ($CHCl_3$) 3588, 3407, 1616 and 1579 $cm^{-1}$.

HRMS (m/e) 404.3247 (M⁺̇, Calcd. for $C_{26}H_{44}O_3$: 404.3279), 386, 301, 233, 187, 161 and 147 (100%).

*Analysis:*

| | | |
|---|---|---|
| Anal. Calcd. for $C_{26}H_{44}O_3$: | C, 77.17; | H, 10.96. |
| Found: | C, 77.41; | H, 10.77 |

Diastereomer B

Using the procedure of Example 5, 350 mg. (0.709 mmole) of diastereomer B of *cis*-3-[2-benzyloxy-4-(1,1-dimethylheptyl)phenyl]-*trans*-4-(3-hydroxy-2-methylpropyl)-*cis*-5-methylcyclohexanol (product of Example 40) gave 255 mg. (89%) of the title compound.

MP 118—120°C. (diisopropyl ether-hexane).

HRMS (m/e) 404.3237 ($M^{+}$, Calcd. for $C_{26}H_{44}O_3$: 404.3279), 386, 318, 301 (100%), 233, 187, 167 and 147.

*Analysis:*

| | | |
|---|---|---|
| Anal. Calcd. for $C_{26}H_{44}O_3$: | C, 77.17; | H, 10.96. |
| Found: | C, 77.40; | H, 10.76. |

By means of this procedure, the remaining benzyl ethers of Example 40 are converted to the corresponding phenols.

**Claims for the Contracting States: BE CH DE FR GB IT LI LU NL SE**

1. A 3-phenyl-cycloalkanol derivative having the formula:

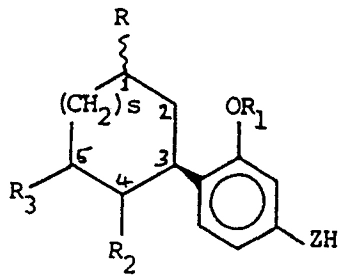

--- (I)

wherein

R is hydroxy or acetamido;

$R_1$ is hydrogen;

$R_2$ is ω-hydroxy($C_{2-4}$)alkyl or ω-methoxy($C_{2-4}$)alkyl;

$R_3$ is hydrogen or methyl;

ZH is alkyl having 7 to 11 carbon atoms; and

s is 1 or 2.

2. A compound as claimed in claim 1, in which R is in *cis*-relationship to the 3-phenyl moiety and $R_2$ is in *trans*-relationship to the 3-phenyl moiety.

3. A compound as claimed in claim 1 or claim 2, in which $R_2$ is ω-hydroxy($C_{2-4}$)alkyl.

4. A compound as claimed in claim 3 in which $R_2$ is 3-hydroxypropyl.

5. A compound as claimed in any preceding claim, in which ZH is a 1,1-dimethylheptyl group.

6. A compound as claimed in claim 5 having the formula:

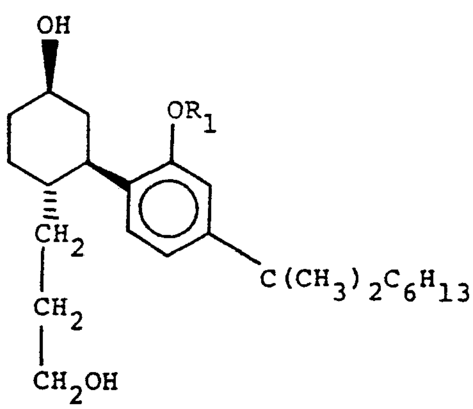

or

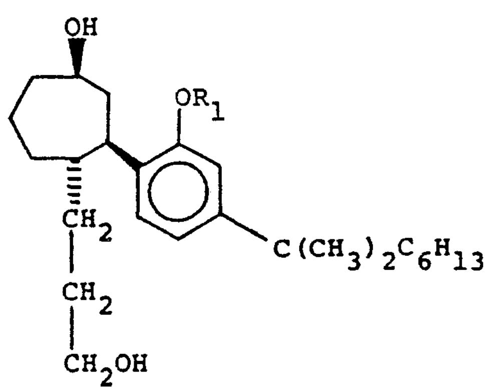

where $R_1$ is hydrogen.

7. A pharmaceutical composition comprising a compound as claimed in any preceding claim, together with a pharmaceutically acceptable carrier material.

8. A derivative of a compound as claimed in claim 1, in which *either* R is mandeloyloxy, *and/or* $R_1$ is benzyl *and/or* $R_2$ is ω-mandeloyloxy($C_{2-4}$)alkyl.

9. A derivative of a compound as claimed in claim 6, in which $R_1$ is benzyl.

10. A diastereomeric bis-mandelate of a compound as claimed in claim 6 or claim 9.

**Claims for the Contracting State: AT**

1. A process for preparing a compound of the formula:

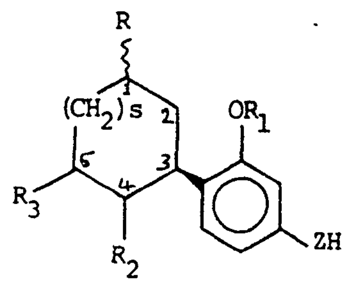

--- (I)

wherein

R is hydroxy or acetamido;

$R_1$ is hydrogen;

$R_2$ is ω-hydroxy($C_{2-4}$)alkyl or ω-methoxy($C_{2-4}$)alkyl;

$R_3$ is hydrogen or methyl;

ZH is alkyl having 7 to 11 carbon atoms; and

s is 1 or 2,

or a derivative thereof in which *either* R is mandeloyloxy *and/or* $R_1$ is benzyl *and/or* $R_2$ is ω-mandeloyloxy($C_{2-4}$)alkyl, which comprises reducing a compound of the formula:

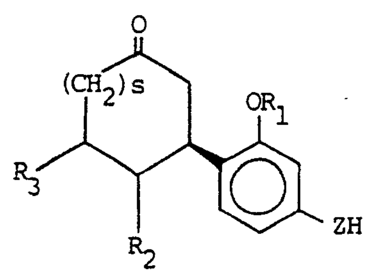

--- (II)

or a precursor thereof, to form a compound of formula I in which R is hydroxy and, if desired, acylating to form a compound in which R is mandeloyloxy and/or $R_2$ is ω-mandeloyloxy($C_{2-4}$)alkyl.

2. A process according to claim 1, in which the precursor is a compound of formula II in which $R_2$ is replaced by a ($C_{2-4}$)alkenyl group which is subsequently hydrated to form an ω-hydroxy($C_{2-4}$)alkyl group.

3. A process according to claim 1, in which the precursor is a compound of formula II in which $R_2$ is replaced by an ω-benzyloxy($C_{2-4}$)alkyl group which is subsequently debenzylated to form an ω-hydroxy($C_{2-4}$)alkyl group.

4. A process according to any preceding claim, in which the precursor is a compound of formula II in which ZH is replaced by an alkenyl group of 7 to 11 carbon atoms, which is subsequently hydrogenated to form an alkyl group of 7 to 11 carbon atoms.

**Patentansprüche für die Vertragsstaaten: BE CH DE FR GB IT LI LU NL SE**

1. Ein 3-Phenylcycloalkanol-derivat mit der Formel

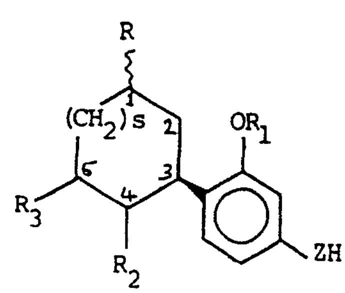

--- (I)

worin

R Hydroxy oder Acetamido ist,

$R_1$ Wasserstoff ist;

$R_2$ ω-Hydroxy($C_{2-4}$)alkyl oder ω-Methoxy($C_{2-4}$)alkyl ist;

$R_3$ Wasserstoff oder Methyl ist;

ZH Alkyl mit 7 bis 11 Kohlenstoffatomen ist; und
s = 1 oder 2 ist.

2. Eine Verbindung gemäß Anspruch 1, in welcher R in cis-Beziehung zum 3-Phenylrest und $R_2$ in trans-Beziehung zum 3-Phenylrest steht.

3. Eine Verbindung gemäß Anspruch 1 oder Anspruch 2, in welcher $R_2$ ω-Hydroxy($C_{2-4}$)alkyl ist.

4. Eine Verbindung gemäß Anspruch 3, in welcher $R_2$ 3-Hydroxypropyl ist.

5. Eine Verbindung gemäß irgendeinem der vorhergehenden Ansprüche, in welcher ZH eine 1,1-Dimethylheptylgruppe ist.

6. Eine Verbindung gemäß Anspruch 5 mit der Formel

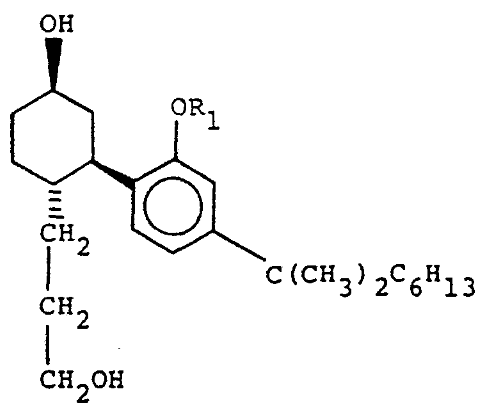

or

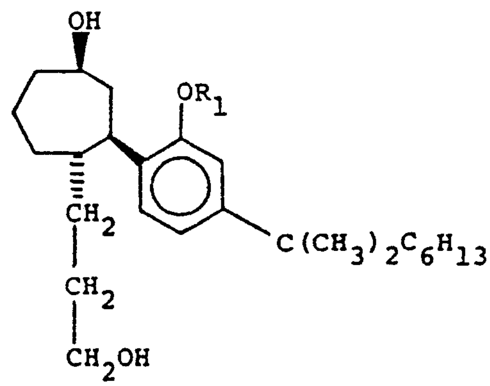

worin $R_1$ Wasserstoff ist.

7. Eine pharmazeutische Zusammensetzung, umfassend eine Verbindung gemäß irgendeinem vorhergehenden Anspruch, zusammen mit einem pharmazeutisch annehmbaren Trägermaterial.

8. Ein Derivat einer Verbindung gemäß Anspruch 1, in welcher entweder R Mandeloyloxy und/oder $R_1$ Benzyl und/oder $R_2$ ω-Mandeloyloxy($C_{2-4}$)alkyl ist.

9. Ein Derivat einer Verbindung gemäß Anspruch 6, in welcher $R_1$ Benzyl ist.

10. Ein diastereomeres Bis-mandelat einer Verbindung gemäß Anspruch 6 oder Anspruch 9.

**Patentansprüche für den Vertragsstaat: AT**

1. Ein Verfahren zur Herstellung einer Verbindung der Formel

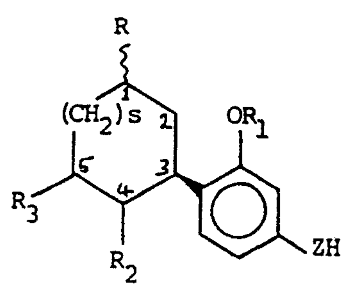

--- (I)

worin
R Hydroxy oder Acetamido ist;
$R_1$ Wasserstoff ist;
$R_2$ ω-Hydroxy($C_{2-4}$)alkyl oder ω-Methoxy($C_{2-4}$)alkyl ist;
$R_3$ Wasserstoff oder Methyl ist;
ZH Alkyl mit 7 bis 11 Kohlenstoffatomen ist; und
s = 1 oder 2 ist,
oder eines Derivates derselben, in welchem entweder R Mandeloyloxy und/oder $R_1$ Benzyl und/oder $R_2$ ω-Mandeloyloxy($C_{2-4}$)alkyl ist, dadurch gekennzeichnet, daß man eine Verbindung der Formel

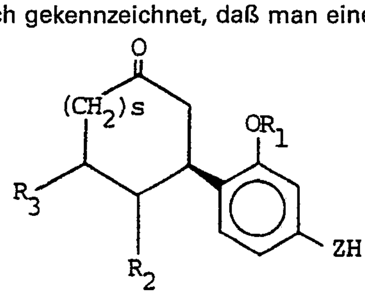

--- (II)

oder einen Vorläufer derselben zur Bildung einer Verbindung der Formel I, in welcher R Hydroxy ist, reduziert und gegebenenfalls zur Bildung einer Verbindung, in welcher R Mandeloyloxy und/oder $R_2$ ω-Mandeloyloxy($C_{2-4}$)alkyl ist, acyliert.

2. Ein Verfahren gemäß Anspruch 1, in welchem der Vorläufer eine Verbindung der Formel II ist, in welcher $R_2$ durch eine ($C_{2-4}$)Alkenylgruppe ersetzt ist, welche anschließend zur Bildung einer ω-Hydroxy($C_{2-4}$)alkylgruppe hydratisiert wird.

3. Ein Verfahren gemäß Anspruch 1, in welchem der Vorläufer eine Verbindung der Formel II ist, in welcher $R_2$ durch eine ω-Benzyloxy($C_{2-4}$)alkylgruppe ersetzt ist, welche anschließend zur Bildung einer ω-Hydroxy($C_{2-4}$)alkylgruppe debenzyliert wird.

4. Ein Verfahren gemäß irgendeinem vorhergehenden Anspruch, in welchem der Vorläufer eine Verbindung der Formel II ist, in welcher ZH durch eine Alkenylgruppe mit 7 bis 11 Kohlenstoffatomen ersetzt ist, welche anschließend zur Bildung einer Alkylgruppe mit 7 bis 11 Kohlenstoffatomen hydriert wird.

**Revendications pour les Etats contractants: BE CH DE FR GB IT LI LU NL SE**

1. Dérivé 3-phénylcycloalcanol répondant à la formule:

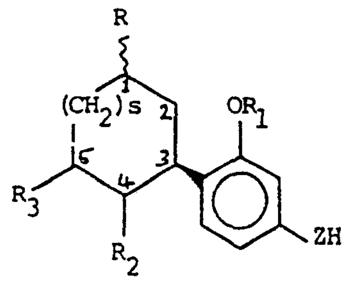

--- (I)

dans laquelle
R est un groupe hydroxy ou acétamido;
$R_1$ représente l'hydrogène;
$R_2$ est un groupe ω-hydroxyalkyle en $C_{2-4}$ ou ω-méthoxyalkyle en $C_{2-4}$;
$R_3$ représente l'hydrogène ou un groupe méthyle;
ZH est un groupe alkyle ayant de 7 à 11 atomes de carbone; et
s représente 1 ou 2.

2. Composé selon la revendication 1 dans lequel R est en relation cis par rapport au motif 3-phényle et $R_2$ est en relation trans par rapport au motif 3-phényle.

3. Composé selon la revendication 1 ou 2, dans lequel $R_2$ est un radical ω-hydroxyalkyle en $C_{2-4}$.

4. Composé selon la revendication 3, dans lequel $R_2$ est un groupe 3-hydroxypropyle.

5. Composé selon l'une quelconque des revendications précédentes, dans lequel ZH est un groupe 1,1-diméthylheptyle.

6. Composé selon la revendication 5 répondant à la formule:

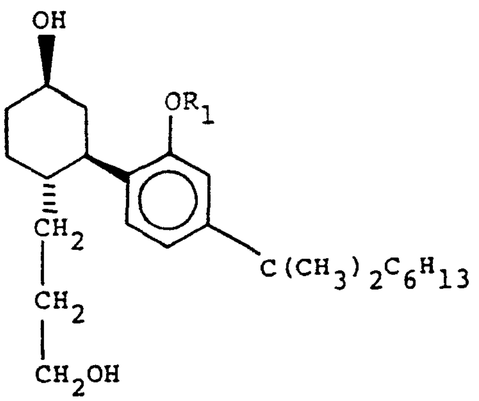

or

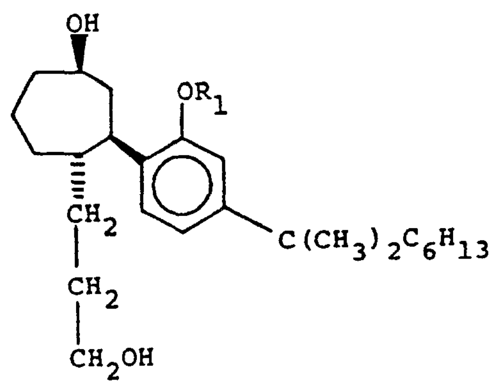

dans laquelle $R_1$ représente l'hydrogène.

7. Composition pharmaceutique comprenant un composé selon l'une quelconque des revendications précédentes avec un matériau support pharmaceutiquement acceptable.

8. Dérivé d'un composé selon la revendication 1, dans lequel ou R est un groupe mandeloyloxy, et/ou $R_1$ est un groupe benzyle et/ou $R_2$ est un groupe ω-mandeloyloxyalkyle en $C_{2-4}$.

9. Dérivé d'un composé selon la revendication 6, dans lequel $R_1$ est un groupe benzyle.

10. Bis-mandélate diastéréomère d'un composé selon la revendication 6 ou la revendication 9.

**Revendications pour l'Etat contractant: AT**

1. Procédé de préparation d'un composé de formule:

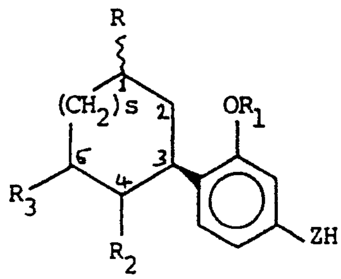

--- (I)

dans laquelle

R est un groupe hydroxy ou acétamido;

$R_1$ représente l'hydrogène;

$R_2$ est un groupe ω-hydroxyalkyle en $C_{2-4}$ ou ω-méthoxyalkyle en $C_{2-4}$;

$R_3$ représente l'hydrogène ou un groupe méthyle;

ZH est un groupe alkyle ayant de 7 à 11 atomes de carbone; et

s représente 1 ou 2,

ou un dérivé d'un tel composé dans lequel ou R est un groupe mandéloyloxy, et/ou $R_1$ est un groupe benzyle et/ou $R_2$ est un groupe ω-mandéloyloxyalkyle en $C_{2-4}$, qui consiste à réduire un composé de formule:

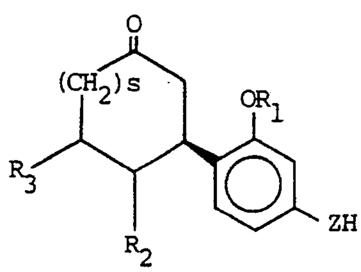

--- (II)

ou un précurseur d'un tel composé, pour former un composé de formule (I) dans lequel $R_1$ est un groupe hydroxy et, si on le désire, à acyler ce composé pour former un composé dans lequel R est un groupe mandéloyloxy et/ou $R_2$ est un groupe ω-mandéloyloxyalkyle en $C_{2-4}$.

2. Procédé selon la revendication 1, dans lequel le précurseur est un composé de formule (II) dans laquelle $R_2$ est remplacé par un groupe alkényle en $C_{2-4}$ qui est ultérieurement hydraté pour former un groupe ω-hydroxy alkyle en $C_{2-4}$.

3. Procédé selon la revendication 1, dans lequel le précurseur est un composé de formule (II) dans laquelle $R_2$ est remplacé par un groupe ω-benzyloxyalkyle en $C_{2-4}$ qui est ultérieurement débenzylé pour former un groupe ω-hydroxyalkyle en $C_{2-4}$.

4. Procédé selon l'une quelconque des revendications précédentes, dans lequel le précurseur est un composé de formule (II) dans lequel ZH est remplacé par un groupe alkényle ayant de 7 à 11 atomes de carbone, qui est ultérieurement hydrogéné pour former un groupe alkyle de 7 à 11 atomes de carbone.